Europäisches Patentamt
European Patent Office
Office européen des brevets

(19) (11) Veröffentlichungsnummer: **0 024 309**
**A2**

(12) # EUROPÄISCHE PATENTANMELDUNG

(21) Anmeldenummer: **80104300.1**

(22) Anmeldetag: **22.07.80**

(51) Int. Cl.³: **C 07 D 487/04**, C 07 D 249/12, A 61 K 31/50 // (C07D487/04, 249/00, 237/00)

(30) Priorität: **23.07.79 GB 7925576**
**22.04.80 GB 8013203**

(43) Veröffentlichungstag der Anmeldung: **04.03.81**
**Patentblatt 81/9**

(84) Benannte Vertragsstaaten: **AT BE CH DE FR GB IT LI LU NL SE**

(71) Anmelder: **F. HOFFMANN-LA ROCHE & CO. Aktiengesellschaft, CH-4002 Basel (CH)**

(72) Erfinder: **Hassall, Cedric Herbert, 23 The Ryde, Hatfield, Herts. AL9 5 DL (GB)**
Erfinder: **Moody, Christopher John, 3 Damask Close, Weston Stevenage, Herts. (GB)**

(74) Vertreter: **Lederer, Franz, Dr. et al, Patentanwälte Dr. Franz Lederer Reiner F. Meyer Lucile-Grahn-Strasse 22, D-8000 München 80 (DE)**

(54) **Triazolopyridazinderivate, Verfahren und Zwischenprodukte zu ihrer Herstellung, diese enthaltende Arzneimittel und deren therapeutische Verwendung.**

(57) Neue Verbindungen der allgemeinen Formel

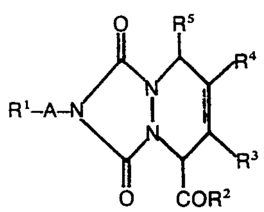

I

worin A eine gegebenenfalls durch niederes Alkyl substituierte Methylen-, Aethylen- oder Propylen Gruppe bedeutet, $R^1$ Halogen, Carboxyl, niederes Alkoxycarbonyl, Aminocarbonyl, Hydroxyaminocarbonyl, Hydrazinocarbonyl, Mercapto, niederes Alkanoylthio oder Aryl-(niederes Alkylthio) und $R^2$ Hydroxy, niederes Alkoxy oder Amino bedeuten, und entweder $R^3$ und $R^4$ Wasserstoff oder Halogen bedeuten, oder $R^3$ Wasserstoff und $R^4$ Hydroxy bedeuten, $R^5$ Wasserstoff, niederes Alkyl, Aryl, Carboxy, niederes Alkoxycarbonyl oder Aminocarbonyl bedeutet und, wenn $R^3$ und $R^4$ beide Wasserstoff bedeuten, die gestrichelte Linie eine fakultative Kohlenstoff-Kohlenstoff-Bindung bedeutet, und Salze von Carbonsäuren der allgemeinen Formel I mit pharmazeutisch annehmbaren Basen, ihre Herstellung sowie pharmazeutische Präparate enthaltend Verbindungen der allgemeinen Formel I oder Salze davon werden beschrieben. Diese Verbindungen und Salze besitzen antihypertensive Wirkungen.

EP 0 024 309 A2

22. Juli 1980 0024309

F.Hoffmann-La Roche & Co. Aktiengesellschaft,Basel,Schweiz

RAN 4019/83

# Triazolopyridazinderivate, Verfahren und Zwischenprodukte zu ihrer Herstellung, diese enthaltende Arzneimittel und deren therapeutische Verwendung

Die vorliegende Erfindung betrifft Triazolopyridazin-Derivate. Im speziellen betrifft die vorliegende Erfindung Triazolopyridazin-Derivate, ein Verfahren zur Herstellung von solchen Verbindungen und pharmazeutische Präparate enthaltend solche Verbindungen.

Die Triazolopyridazin-Derivate, gemäss vorliegender Erfindung, sind Verbindungen der allgemeinen Formel

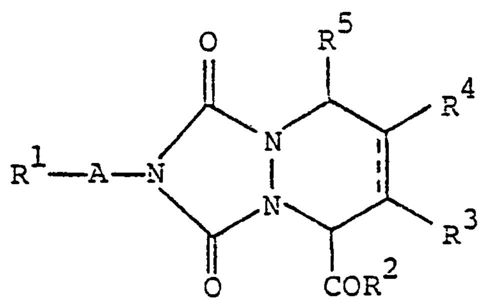

I

worin A eine gegebenenfalls durch niederes Alkyl substituierte Methylen-, Aethylen- oder Propylen-Gruppe bedeutet, $R^1$ Halogen, Carboxyl, niederes Alkoxycarbonyl, Aminocarbonyl, Hydroxyaminocarbonyl, Hydrazinocarbonyl, Mercapto, niederes Alkanoylthio oder Aryl-(niederes Alkylthio) und $R^2$ Hydroxy, niederes Alkoxy oder Amino bedeuten, und entweder $R^3$ und $R^4$ Wasserstoff oder Halogen bedeuten, oder

Nt/ 26.6.80

$R^3$ Wasserstoff und $R^4$ Hydroxy bedeuten, $R^5$ Wasserstoff, niederes Alkyl, Aryl, Carboxy, niederes Alkoxycarbonyl oder Aminocarbonyl bedeutet und, wenn $R^3$ und $R^4$ beide Wasserstoff bedeuten, die gestrichelte Linie eine fakultative Kohlenstoff-Kohlenstoff-Bindung bedeutet,

und Salze von Carbonsäuren der allgemeinen Formel I mit pharmazeutisch annehmbaren Basen.

Der Ausdruck "niederes Alkyl", wie er in den vorliegenden Erfindung verwendet wird, alleine oder in Kombinationen bedeutet eine geradkettige oder verzweigte Alkylgruppe, welche vorzugsweise 1-6 Kohlenstoffatome enthält, beispielsweise Methyl, Aethyl, Propyl, Isopropyl, Butyl, t-Butyl, Pentyl, Hexyl und dergleichen. Der Ausdruck "niederes Alkoxy" für sich allein genommen oder in Kombinationen bedeutet geradkettige oder verzweigte Alkoxygruppen, welche vorzugsweise 1-6 Kohlenstoffatome enthalten, beispielsweise Methoxy, Aethoxy, Propoxy, Isopropoxy und dergleichen. Beispiele für niedere Alkoxycarbonyl-Gruppen sind Methoxycarbonyl, Aethoxycarbonyl und dergleichen. Der niedere Alkanoyl-Rest einer niederen Alkanoylthio-Gruppe leitet sich von einer geradkettigen oder verzweigten Fettsäure ab, welche vorzugsweise bis zu 6 Kohlenstoffatomen enthält, wie Essigsäure, Propionsäure, Buttersäure, Isobuttersäure, Valeriansäure, Pivalinsäure und dergleichen; beispielhaft für niedere Alkanoylthio-Gruppen sind demnach Acetylthio, Propionylthio und dergleichen. Der Ausdruck "Aryl" für sich allein genommen oder in Kombinationen bedeutet eine Phenylgruppe oder eine Phenylgruppe welche einen oder mehrere der folgenden Substituenten enthält: Halogen, niederes Alkyl, niederes Alkoxy, Trifluormethyl, Nitro und dergleichen. Als Beispiel für eine Aryl-(niedere Alkylthio)-Gruppe sei die Benzylthio-Gruppe erwähnt. Der Ausdruck "Halogen" bedeutet Fluor, Chlor, Brom oder Jod.

Bevorzugte Verbindungen der allgemeinen Formel I sind

solche, worin A die Aethylengruppe bedeutet. Weitere bevorzugte Verbindungen der allgemeinen Formel I sind solche, worin $R^1$ Carboxyl, Hydroxyaminocarbonyl oder Mercapto bedeutet. Die bevorzugte Bedeutungsmöglichkeit von $R^2$ ist Hydroxy. Verbindungen der allgemeinen Formel I, die in 6,7-Stellung eine Doppelbindung besitzen sind ebenfalls bevorzugt. Die bevorzugte Bedeutungsmöglichkeit von $R^5$ ist Wasserstoff oder Aryl, wobei Phenyl besonders bevorzugt wird.

Besonders bevorzugte Verbindungen der allgemeinen Formel I sind:

5-Carboxy-2,3,5,8-tetrahydro-1,3-dioxo-1H-1,2,4-triazolo[1,2-a]pyridazin-2-essigsäure,
5-Carboxy-2,3,5,8-tetrahydro-1,3-dioxo-1H-1,2,4-triazolo[1,2-a]pyridazin-2-buttersäure,
2-(2-Chloräthyl)-2,3,5,8-tetrahydro-1,3-dioxo-1H-1,2,4-triazolo[1,2-a]pyridazin-5-carbonsäure,
2-(3-Chlorpropyl)-2,3,5,8-tetrahydro-1,3-dioxo-1H-1,2,4-triazolo[1,2-a]pyridazin-5-carbonsäure,
Methyl-5-carboxy-2,3,5,8-tetrahydro-1,3-dioxo-1H-1,2,4-triazolo[1,2-a]pyridazin-2-propionat,
5-Carboxy-2,3,5,8-tetrahydro-8-methyl-1,3-dioxo-1H-1,2,4-triazolo[1,2-a]pyridazin-2-propionsäure,
2-(2-Benzylthioäthyl)-2,3,5,8-tetrahydro-1,3-dioxo-1H-1,2,4-triazolo[1,2-a]pyridazin-5-carbonsäure,
Methyl-2-acetylthiomethyl-2,3,5,8-tetrahydro-1,3-dioxo-1H-1,2,4-triazolo[1,2-a]pyridazin-5-carboxylat,
t-Butyl-2-(2-acetylthioäthyl)-2,3,5,8-tetrahydro-1,3-dioxo-1H-1,2,4-triazolo[1,2-a]pyridazin-5-carboxylat,
t-Butyl-2-[2-(N-hydroxycarbamoyl)äthyl]-2,3,5,8-tetrahydro-1,3-dioxo-1H-1,2,4-triazolo[1,2-a]pyridazin-5-carboxylat,
2-(2-Carbamoyläthyl)-2,3,5,8-tetrahydro-1,3-dioxo-1H-1,2,4-triazolo[1,2-a]pyridazin-5-carbonsäure,
5-Carbamoyl-2,3,5,8-tetrahydro-1,3-dioxo-1H-1,2,4-triazolo[1,2-a]pyridazin-2-propionamid,

2-[(2-Hydrazinocarbonyl)äthyl]-2,3,5,8-tetrahydro-1,3-dioxo-1H-1,2,4-triazolo[1,2-a]pyridazin-5-carbonsäure,

5-Carboxy-2,3,5,6,7,8-hexahydro-1,3-dioxo-1H-1,2,4-triazolo[1,2-a]pyridazin-2-essigsäure,

5-Carboxy-2,3,5,6,7,8-hexahydro-1,3-dioxo-1H-1,2,4-triazolo[1,2-a]pyridazin-2-propionsäure,

5-Carboxy-2,3,5,6,7,8-hexahydro-1,3-dioxo-1H-1,2,4-triazolo[1,2-a]pyridazin-2-buttersäure,

Methyl-2,3,5,6,7,8-hexahydro-1,3-dioxo-1H-1,2,4-triazolo[1,2-a]pyridazin-2-propionat,

2-(2-Chloräthyl)-2,3,5,6,7,8-hexahydro-1,3-dioxo-1H-1,2,4-triazolo[1,2-a]pyridazin-5-carbonsäure,

Benzyl-5-methoxycarbonyl-2,3,5,8-tetrahydro-1,3-dioxo-1H-1,2,4-triazolo[1,2-a]pyridazin-2-acetat,

Methyl-2-(2-chloräthyl)-2,3,5,6,7,8-hexahydro-1,3-dioxo-1H-1,2,4-triazolo[1,2-a]pyridazin-5-carboxylat,

Methyl-2-chlormethyl-2,3,5,8-tetrahydro-1,3-dioxo-1H-1,2,4-triazolo[1,2-a]pyridazin-5-carboxylat,

6,7-Dibrom-5-carboxy-hexahydro-1,3-dioxo-1H-1,2,4-triazolo[1,2-a]pyridazin-2-propionsäure,

5-Carboxy-hexahydro-7-hydroxy-1,3-dioxo-1H-1,2,4-triazolo[1,2-a]pyridazin-2-propionsäure,

t-Butyl-2-(2-chloräthyl)-2,3,5,8-tetrahydro-1,3-dioxo-1H-1,2,4-triazolo[1,2-a]pyridazin-5-carboxylat,

Methyl-2,3,5,8-tetrahydro-5-t-butoxycarbonyl-1,3-dioxo-1H-1,2,4-triazolo[1,2-a]pyridazin-2-propionat und

Methyl-2,3,5,8-tetrahydro-5-methoxycarbonyl-1,3-dioxo-1H-1,2,4-triazolo[1,2-a]pyridazin-2-propionat.

Weitere bevorzugte Verbindungen der allgemeinen Formel I sind:

Dimethyl-2-(2-methoxycarbonyläthyl)-2,3,5,8-tetrahydro-1,3-dioxo-1H-1,2,4-triazolo[1,2-a]pyridazin-5,8-dicarboxylat,

2-(2-Carboxyäthyl)-2,3,5,8-tetrahydro-1,3-dioxo-1H-1,2,4-triazolo[1,2-a]pyridazin-5,8-dicarbonsäure,

cis-2-(2-Chloräthyl)-2,3,5,8-tetrahydro-1,3-dioxo-8-phenyl-1H-1,2,4-triazolo[1,2-a]pyridazin-5-carbonsäure,

Aethyl-cis-2-(2-chloräthyl)-2,3,5,8-tetrahydro-1,3-dioxo-8-phenyl-1H-1,2,4-triazolo[1,2-a]pyridazin-5-carboxylat,

Aethyl-trans-2-(2-chloräthyl)-2,3,5,8-tetrahydro-1,3-dioxo-8-phenyl-1H-1,2,4-triazolo[1,2-a]pyridazin-5-carboxylat,

trans-2-(2-Chloräthyl)-2,3,5,8-tetrahydro-1,3-dioxo-8-phenyl-1H-1,2,4-triazolo[1,2-a]pyridazin-5-carbonsäure,

Methyl-trans-2-(2-acetylthioäthyl)-2,3,5,8-tetrahydro-1,3-dioxo-8-phenyl-1H-1,2,4-triazolo[1,2-a]pyridazin-5-carboxylat,

Methyl-cis-2-(2-acetylthioäthyl)-2,3,5,8-tetrahydro-1,3-dioxo-8-phenyl-1H-1,2,4-triazolo[1,2-a]pyridazin-5-carboxylat,

trans-2,3,5,8-Tetrahydro-2-(2-mercaptoäthyl)-1,3-dioxo-8-phenyl-1H-1,2,4-triazolo[1,2-a]pyridazin-5-carbonsäure,

Aethyl-2,3,5,6,7,8-hexahydro-2-(2-chloräthyl)-1,3-dioxo-8-phenyl-1H-1,2,4-triazolo[1,2-a]pyridazin-5-carboxylat,

Aethyl-2,3,5,6,7,8-hexahydro-2-(2-acetylthioäthyl)-1,3-dioxo-8-phenyl-1H-1,2,4-triazolo[1,2-a]pyridazin-5-carboxylat,

2,3,5,6,7,8-Hexahydro-2-(2-mercaptoäthyl)-1,3-dioxo-8-phenyl-1H-1,2,4-triazolo[1,2-a]pyridazin-5-carbonsäure,

Aethyl-2-(2-chloräthyl)-2,3,5,8-tetrahydro-8-(4-chlorphenyl)-1,3-dioxo-1H-1,2,4-triazolo[1,2-a]pyridazin-5-carboxylat,

Aethyl-2-(2-chloräthyl)-2,3,5,6,7,8-hexahydro-8-(4-chlorphenyl)-1,3-dioxo-1H-1,2,4-triazolo[1,2-a]pyridazin-5-carboxylat,

Aethyl-2-(2-acetylthioäthyl)-2,3,5,6,7,8-hexahydro-8-(4-chlorphenyl)-1,3-dioxo-1H-1,2,4-triazolo[1,2-a]-pyridazin-5-carboxylat,

8-(4-Chlorphenyl)-2,3,5,6,7,8-hexahydro-2-(2-mercaptoäthyl)-1,3-dioxo-1H-1,2,4-triazolo[1,2-a]-pyridazin-5-carbonsäure,

Aethyl-cis-2-(2-chloräthyl)-2,3,5,8-tetrahydro-8-(4-methoxyphenyl)-1,3-dioxo-1H-1,2,4-triazolo[1,2-a]-pyridazin-5-carboxylat,

cis-2-(2-Chloräthyl)-2,3,5,8-tetrahydro-8-(4-methoxyphenyl)-1,3-dioxo-1H-1,2,4-triazolo[1,2-a]-pyridazin-5-carbonsäure,

Aethyl-cis-2-(2-chloräthyl)-2,3,5,8-tetrahydro-8-(4-nitrophenyl)-1,3-dioxo-1H-1,2,4-triazolo[1,2-a]-pyridazin-5-carboxylat,

cis-2-(2-Chloräthyl)-2,3,5,8-tetrahydro-8-(4-nitrophenyl)-1,3-dioxo-1H-1,2,4-triazolo[1,2-a]pyridazin-5-carbonsäure,

Aethyl-2-chlormethyl-1,3,5,8-tetrahydro-1,3-dioxo-8-phenyl-1H-1,2,4-triazolo[1,2-a]pyridazin-5-carboxylat,

Aethyl-2-chlormethyl-2,3,5,6,7,8-hexahydro-1,3-dioxo-8-phenyl-1H-1,2,4-triazolo[1,2-a]pyridazin-5-carboxylat,

Aethyl-2-acetylthiomethyl)-2,3,5,6,7,8-hexahydro-1,3-dioxo-8-phenyl-1H-1,2,4-triazolo[1,2-a]pyridazin-5-carboxylat,

2,3,5,6,7,8-Hexahydro-2-mercaptomethyl-1,3-dioxo-8-phenyl-1H-1,2,4-triazolo[1,2-a]pyridazin-5-carbonsäure,

2,3,5,8-Tetrahydro-2-mercaptomethyl-1,3-dioxo-1H-1,2,4-triazolo[1,2-a]pyridazin-5-carbonsäure,

Methyl-2,3,5,8-tetrahydro-2-(3-acetylthiopropyl)-1,3-dioxo-1H-1,2,4-triazolo[1,2-a]pyridazin-5-carboxylat,

2,3,5,8-Tetrahydro-2-(3-mercaptopropyl)-1,3-dioxo-1H-1,2,4-triazolo[1,2-a]pyridazin-5-carbonsäure,

Methyl-2-chlormethyl-2,3,5,6,7,8-hexahydro-1,3-dioxo-1H-1,2,4-triazolo[1,2-a]pyridazin-5-carboxylat,

Methyl-2-acetylthiomethyl)-2,3,5,6,7,8-hexahydro-1,3-dioxo-1H-1,2,4-triazolo[1,2-a]pyridazin-5-carbonsäure und

2,3,5,6,7,8-Hexahydro-2-mercaptomethyl-1,3-dioxo-1H-1,2,4-triazolo[1,2-a]pyridazin-5-carbonsäure.

Besonders bevorzugte von der allgemeinen Formel I umfasste Verbindungen sind:

5-Carboxy-2,3,5,8-tetrahydro-1,3-dioxo-1H-1,2,4-triazolo[1,2-a]pyridazin-2-propionsäure,

5-Carboxy-2,3,5,8-tetrahydro-8-phenyl-1,3-dioxo-1H-1,2,4-triazolo[1,2-a]pyridazin-2-propionsäure,

2,3,5,8-Tetrahydro-2-[2-mercaptoäthyl)-1,3-dioxo-1H-1,2,4-triazolo[1,2-a]pyridazin-5-carbonsäure und

2-[2-(N-Hydroxycarbamoyl)äthyl]-2,3,5,8-tetrahydro-1,3-dioxo-1H-1,2,4-triazolo[1,2-a]pyridazin-5-carbonsäure.

Die neuen Triazolopyridazin-Derivate der allgemeinen Formel I und Salze von Carbonsäuren der allgemeinen Formel I mit pharmazeutisch annehmbaren Basen können erfindungsgemäss hergestellt werden, indem man

(a) zur Herstellung von Verbindungen der allgemeinen Formel I, worin $R^1$ Halogen, Carboxyl oder niederes Alkoxycarbonyl bedeutet, und in 6,7-Stellung eine Doppelbindung vorhanden ist und A, $R^2$ und $R^5$ obige Bedeutung besitzen, eine Verbindung der allgemeinen Formel

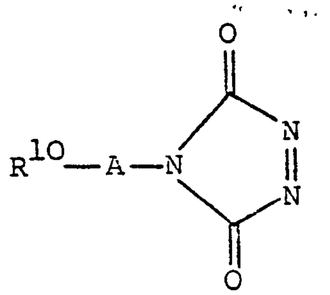

II

worin $R^{10}$ Halogen, Carboxyl oder niederes Alkoxycarbonyl bedeutet,

mit einer Verbindung der allgemeinen Formel

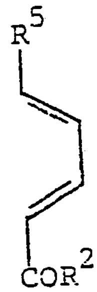

III

worin $R^2$ und $R^5$ obige Bedeutung besitzen, umsetzt, oder

(b) zur Herstellung von Verbindungen der allgemeinen Formel I, worin $R^1$ niederes Alkanoylthio oder Aryl-(niederes Alkylthio) bedeutet und A, $R^2$, $R^3$, $R^4$, $R^5$ und die gestrichelte Linie obige Bedeutung besitzen, eine Verbindung der allgemeinen Formel

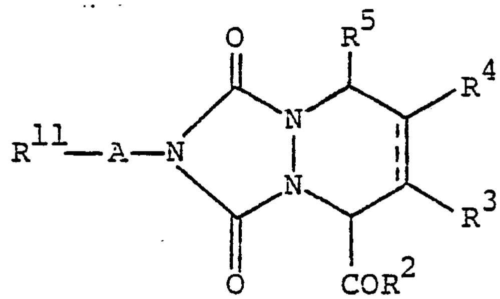

Ia

worin A, $R^2$, $R^3$, $R^4$, $R^5$ und die gestrichelte Linie obige Bedeutung besitzen, und $R^{11}$ Halogen bedeutet,

mit einer Verbindung der allgemeinen Formel

$$R^6\text{-SH} \qquad \text{IV}$$

worin $R^6$ niederes Alkanoyl oder Aryl-(niederes Alkyl) bedeutet,

umsetzt, oder

(c) zur Herstellung von Verbindungen der allgemeinen Formel I, worin $R^1$ Mercapto bedeutet, und A, $R^2$, $R^3$, $R^4$, $R^5$ und die gestrichelte Linie obige Bedeutung besitzen, in einer Verbindung der allgemeinen Formel

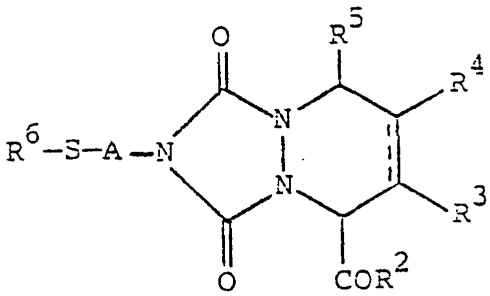

Ib

worin A, $R^2$, $R^5$, $R^6$ und die gestrichelte Linie obige Bedeutung besitzen,

die mit $R^6$ bezeichnete Gruppe abspaltet, oder

(d) zur Herstellung von Verbindungen der allgemeinen Formel I, worin $R^1$ Aminocarbonyl, Hydroxyaminocarbonyl oder Hydrazinocarbonyl bedeutet, und A, $R^2$, $R^3$, $R^4$, $R^5$ und die gestrichelte Linie obige Bedeutung besitzen, eine Verbindung der allgemeinen Formel

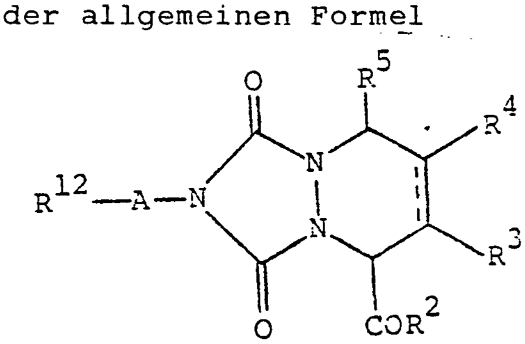

Ic

worin A, $R^2$, $R^3$, $R^4$, $R^5$ und die gestrichelte Linie obige Bedeutung besitzen, und $R^{12}$ niederes Alkoxycarbonyl bedeutet,

mit einer Verbindung der allgemeinen Formel

$$Y\text{-}NH_2 \qquad V$$

worin Y Wasserstoff, Hydroxy oder Amino bedeutet,

umsetzt, oder

(e) zur Herstellung von Verbindungen der allgemeinen Formel I, worin $R^1$ Halogen, Carboxyl, niederes Alkoxycarbonyl, Aminocarbonyl, Hydroxyaminocarbonyl oder Hydrazinocarbonyl, $R^3$ und $R^4$ beide Wasserstoff bedeuten, in 6,7-

0024309

Stellung eine Einfachbindung vorhanden ist, und A, $R^2$ und $R^5$ obige Bedeutung besitzen, eine entsprechende Verbindung der allgemeinen Formel I, worin in 6,7-Stellung eine Doppelbindung vorhanden ist, katalytisch hydriert, oder

(f) eine Verbindung der allgemeinen Formel

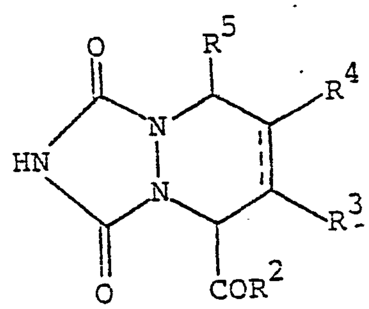

VI

worin $R^2$, $R^3$, $R^4$, $R^5$ und die gestrichelte Linie obige Bedeutung besitzen,

mit einer Verbindung der allgemeinen Formel

$$R^1\text{-A-X} \qquad \text{VII}$$

worin $R^1$ und A obige Bedeutung besitzen und X eine Abgangsgruppe bedeutet,

umsetzt, oder

(g) zur Herstellung von Verbindungen der allgemeinen Formel I, worin A Methylen, $R^1$ Halogen und $R^2$ niederes Alkoxy oder Amino bedeuten und $R^3$, $R^4$, $R^5$ und die gestrichelte Linie obige Bedeutung besitzen, eine Verbindung der allgemeinen Formel

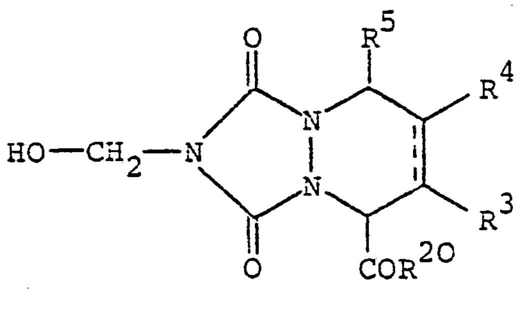

VIII

worin $R^3$, $R^4$, $R^5$ und die gestrichelte Linie obige Bedeutung besitzen, und $R^{20}$ niederes Alkoxy oder Amino bedeutet,

geeignet halogeniert, oder

(h) zur Herstellung von Verbindungen der allgemeinen Formel I, worin $R^3$ und $R^4$ beide Halogen bedeuten, und in 6,7-Stellung eine Einfachbindung vorhanden ist, und A, $R^1$, $R^2$ und $R^5$ obige Bedeutung besitzen, eine Verbindung der allgemeinen Formel I, worin in 6,7-Stellung eine Doppelbindung vorhanden ist, mit einem Halogenwasserstoff in Gegenwart von Wasserstoffperoxid behandelt, oder

(i) zur Herstellung von Verbindungen der allgemeinen Formel I, worin $R^3$ Wasserstoff und $R^4$ Hydroxy bedeuten, und in 6,7-Stellung eine Einfachbindung vorhanden ist, und A, $R^1$, $R^2$ und $R^5$ obige Bedeutung besitzen, eine Verbindung der allgemeinen Formel

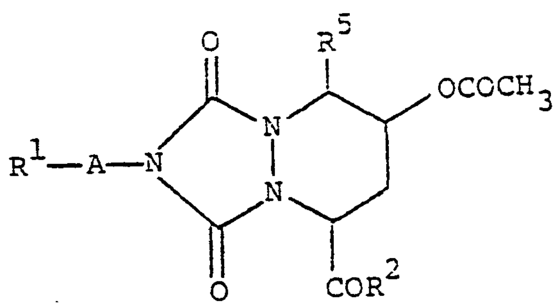

XI

worin A, $R^1$, $R^2$ und $R^5$ obige Bedeutung besitzen,

hydrolysiert, oder

(j) zur Herstellung von Verbindungen der allgemeinen Formel I, worin $R^1$ niederes Alkoxycarbonyl und/oder $R^2$ niederes Alkoxy bedeutet, eine entsprechende Verbindung der allgemeinen Formel I, worin $R^1$ Carboxyl und/oder $R^2$ Hydroxy bedeuten, verestert, oder

(k) zur Herstellung von Verbindungen der allgemeinen Formel I, worin $R^1$ Carboxyl und/oder $R^2$ Hydroxy bedeutet,

eine entsprechende Verbindung der allgemeinen Formel I, worin $R^1$ niederes Alkoxycarbonyl und/oder $R^2$ niederes Alkoxy bedeutet, mit einer Säure oder einer Base behandelt, oder

(l) zur Herstellung eines Salzes einer Carbonsäure der allgemeinen Fomel I, eine Carbonsäure der allgemeinen Formel I in ein pharmazeutisch annehmbares Salz mit einer Base überführt.

Die Reaktion einer Verbindung der allgemeinen Formel II mit einer Verbindung der allgemeimen Formel III, gemäss Variante (a) des erfindungsgemässen Verfahrens, wird zweckmässigerweise in einem inerten organischen Lösungsmittel durchgeführt. Geeignete inerte organische Lösungsmittel umfassen aromatische Kohlenwasserstoffe, wie Benzol, Toluol und dergleichen, halogenierte Kohlenwasserstoffe, wie Methylenchlorid, Chloroform, Chlorbenzol und dergleichen, Aether, wie Diäthyläther, Dioxan und dergleichen, Essigester und dergleichen. Dioxan ist das bevorzugte inerte organische Lösungsmittel. Die Reaktion kann in einem Temperaturbereich von etwa -10°C bis zum Siedepunkt der Reaktionsmischung durchgeführt werden, wobei Raumtemperatur bevorzugt wird. In einer bevorzugten Ausführungsform des vorliegenden Aspektes lässt man Verbindungen der allgemeinen Formel II mit Verbindungen der allgemeinen Formel III in situ reagieren; d.h. ohne Isolierung aus dem Medium in dem sie vorher hergestellt wurden.

Die Reaktion einer Verbindung der allgemeinen Formel Ia mit einer Verbindung der allgemeinen Formel IV, gemäss Variante (b) des erfindungsgemässen Verfahrens, wird vorzugsweise in einem inerten Lösungsmittel in Gegenwart einer Base durchgeführt. Für den vorliegenden Aspekt geeignete Basen sind Alkalimetallhydroxide, wie Natriumhydroxid und Kaliumhydroxid, Alkalimetallhydride, wie Natriumhydrid und Kaliumhydrid, Alkalimetall-niedere Alkoxide,

wie Natriummhoxid, Natriumäthoxid und dergleichen, und Alkalimetallcarbonate, wie Natriumcarbonat und Kaliumcarbonat. Geeignete Lösungsmittel sind, wenn $R^6$ in Verbindungen der allgemeinen Formel IV niederes Alkanoyl bedeutet, di-(niedere Alkyl)-Ketone wie Aceton, und Dimethylformamid oder, wenn ein Alkalimetallcarbonat als Base verwendet wird, eine Mischung aus Wasser und einem chlorierten Kohlenwasserstoff, wie Methylenchlorid, oder eine Mischung aus Wasser und Essigester. Bedeutet $R^6$ in Verbindungen der allgemeinen Formel IV Aryl-(niederes Alkyl), so sind Wasser, Dimethylformamid und dergleichen, geeignete Lösungsmittel. Zweckmässigerweise kann man eine Verbindung der allgemeinen Formel III, worin $R^6$ niederes Alkanoyl bedeutet, als Alkalimetallsalz, beispielsweise als Kaliumsalz, einsetzen und die Reaktion in Gegenwart einer katalytischen Menge eines Alkalimetalljodids, wie Kaliumjodid, durchführen. Die Reaktion einer Verbindung der allgemeinen Formel Ia mit einer Verbindung der allgemeinen Formel IV kann in einem Temperaturbereich von etwa 10°C bis zur Rückflusstemperatur der Reaktionsmischung durchgeführt werden, wobei Siedetemperatur des Reaktionsgemisches bevorzugt wird.

Die Abspaltung der mit $R^6$ bezeichneten Gruppe in einer Verbindung der allgemeinen Formel Ib, gemäss Variante (c) des erfindungsgemässen Verfahrens, kann in an sich bekannter Weise durchgeführt werden. Dabei richtet sich die verwendete Methode nach der Natur der Gruppe $R^6$. Beispielsweise kann man, wenn $R^6$ niederes Alkanoyl bedeutet, die Abspaltung mit wässriger Alkalimetall-Hydroxidlösung, wie wässrige Natronlauge oder wässrige Kalilauge, mit wässrigem Ammoniak, mit einem niederen Alkanol, wie Methanol, in Gegenwart des entsprechenden Alkalimetallalkoxid, beispielsweise Natriummethoxid, oder mit einer Mineralsäure, wie Salzsäure, bei etwa 100°C durchführen. Vorzugsweise verwendet man wässriges Ammoniak. Bedeutet die Gruppe $R^6$ Aryl-(niederes Alkyl), so kann man die Abspaltung beispielsweise mit Natrium in flüssigem Ammoniak durchführen.

Gemäss Verfahrensvariante (d) des erfindungsgemässen Verfahrens kann eine Verbindung der allgemeinen Formel Ic mit einer Verbindung der allgemeinen Formel V, beispielsweise Ammoniak, Hydroxylamin oder Hydrazin, umgesetzt werden. Die Reaktion wird dabei zweckmässigerweise in einem inerten organischen Lösungsmittel, beispielsweise in einem niederen Alkanol, wie Methanol, durchgeführt. Die Reaktion kann in einem Temperaturbereich von etwa 0°C bis zur Siedetemperatur der Reaktionsmischung durchgeführt werden, wobei Raumtemperatur bevorzugt wird. Hydroxylamin kann als Säureadditionssalz, beispielsweise als Hydrochlorid, eingesetzt werden, wobei man in diesem Fall eine geeignete Base, beispielsweise ein Alkalimetallhydroxid, wie Kaliumhydroxid, benötigt.

Gemäss Verfahrensvariante (e) des erfindungsgemässen Verfahrens kann man eine Verbindung der allgemeinen Formel I, worin in 6,7-Stellung eine Doppelbindung vorhanden ist, katalytisch hydrieren. Geeignete Katalysatoren sind Edelmetalle, wie Palladium, Platin, Ruthenium, Rhodium und Raney Nickel. Der Katalysator kann auch zusammen mit einem geeigneten Träger verwendet werden, beispielsweise Palladium auf Kohle, Rhodium auf Aluminium und dergleichen. Die katalytische Hydrierung kann in einem konventionellen inerten organischen Lösungsmittel wie einem aromatischen Kohlenwasserstoff, beispielsweise Benzol, Toluol, Xylol und dergleichen, einem niederen Alkanol, beispielsweise Methanol, Aethanol und dergleichen oder einem Aether, wie Dioxan und dergleichen, durchgeführt werden. Vorteilhafterweise arbeitet man bei Raumtemperatur und bei Atmosphärendruck.

Für die mit X bezeichnete Abgangsgruppe in einer Verbindung der allgemeinen Formel VII kommt jede herkömmliche Abgangsgruppe in Frage, beispielsweise ein Chlor-, Bromoder Jod-Atom oder eine niedere Alkylsulfonyloxygruppe, wie Mesyloxy, oder eine Arylsulfonyloxy-Gruppe, wie Tosyloxy. X bedeutet vorzugsweise ein Brom-Atom.

Die Reaktion einer Verbindung der allgemeinen Formel VI mit einer Verbindung der allgemeinen Formel VII, gemäss Verfahrensvariante (f) des erfindungsgemässen Verfahrens, wird zweckmässigerweise in einem inerten organischen Lösungsmittel in Gegenwart einer Base durchgeführt. Beispielsweise kann man die Reaktion mit einem Alkalimetallalkoxid, wie Kalium-t-Butoxid, im entsprechenden Alkanol, wie t-Butanol, oder einem Alkalimetallcarbonat, wie Kaliumcarbonat, oder einem Alkalimetallhydrid, wie Natriumhydrid, in Dimethylformamid, durchführen. Die Reaktionstemperatur ist nicht kritisch, man arbeitet aber vorzugsweise bei der Siedetemperatur des Reaktionsgemisches.

Die Halogenierung einer Verbindung der allgemeinen Formel VIII, gemäss Verfahrensvariante (g) des erfindungsgemässen Verfahrens, kann in an sich bekannter Weise erfolgen. Geeignete Halogenierungsmittel sind Phosphortrichlorid, Phosphorpentachlorid, Phosphortribromid und Thionylchlorid. Vorzugsweise verwendet man ein Chlorierungsmittel, wobei Phosphorpentachlorid ganz besonders bevorzugt wird. Die Halogenierung wird zweckmässigerweise in einem Aether, wie Diäthyläther und dergleichen, bei Temperaturen von etwa -10° bis +30°C, vorzugsweise bei Raumtemperatur, durchgeführt.

Gemäss Verfahrensvariante (h) des erfindungsgemässen Verfahrens kann eine Verbindung der allgemeinen Formel I, worin in 6,7-Stellung eine Doppelbindung vorhanden ist, mit einem Halogenwasserstoff in Gegenwart von Wasserstoffperoxid behandelt werden. Bei diesem Verfahrensaspekt verwendet man den Halogenwasserstoff vorzugsweise als wässrige Lösung, wobei wässriger Bromwasserstoff bevorzugt wird. Diese Reaktion wird vorteilhafterweise in einem Temperaturbereich von etwa -10° bis +10°C durchgeführt,

vorzugsweise bei etwa + 5°C.

Gemäss Verfahrensvariante (i) des erfindungsgemässen Verfahrens kann eine Verbindung der allgemeinen Formel XI hydrolysiert werden. Die Hydrolyse kann mit Säure oder mit Base in an sich bekannter Weise erfolgen. Beispielsweise kann man Salzsäure bei erhöhter Temperatur, beispielsweise 100°C, verwenden.

Die Veresterung einer Carbonsäure der allgemeinen Formel I, worin $R^1$ Carboxyl und/oder $R^2$ Hydroxy bedeutet, zu einem Ester der allgemeinen Formel I, worin $R^1$ niederes Alkoxycarbonyl und/oder $R^2$ niederes Alkoxy bedeutet, gemäss Verfahrensvariante (j) des erfindungsgemässen Verfahrens, kann in an sich bekannter Weise erfolgen. Beispielsweise kann die Veresterung durchgeführt werden, in dem man eine Carbonsäure der allgemeinen Formel I mit einem niederen Alkanol, wie Methanol, Aethanol oder dergleichen, in Gegenwart einer geeigneten Säure, wie einer Mineralsäure, wie Salzsäure, oder mit einem geeigneten Diazoalkan, wie Diazomethan, durchgeführt werden. Andererseits kann man eine Carbonsäure der allgemeinen Formel I zuerst in an sich bekannter Weise in das entsprechende Säurechlorid überführen beispielsweise durch Behandeln mit Chlorierungsmitteln, wie Tionylchlorid, Phosphortrichlorid oder Phosphorpentachlorid, das dann ebenfalls in an sich bekannter Weise, mit einem niederen Alkanol umgesetzt wird. t-Butylester können ebenso erhalten werden, indem man eine Carbonsäure der allgemeinen Formel I mit Isobuten in Gegenwart von Schwefelsäure umsetzt.

Gemäss Verfahrensvariante (k) des erfindungsgemässen Verfahrens kann ein Ester der allgemeinen Formel I, worin $R^1$ niederes Alkoxycarbonyl und/oder $R^2$ niederes Alkoxy bedeutet, in eine Säure der allgemeinen Formel I, worin $R^1$ Carboxyl und/oder $R^2$ Hydroxy bedeutet, überführt werden. Diese Reaktion kann in an sich bekannter Weise durchgeführt werden; beispielsweise durch Behandeln mit einer wässrigen Alkalimetallhydroxid-Lösung, wie wässrige Natronlauge oder wässrige Kalilauge, oder einer wässrigen Mineralsäure, wie wässrige Salzsäure, zweckmässigerweise in einem Temperaturbereich von etwa Raumtemperatur bis zum Siedepunkt der Reaktionsmischung, oder, wenn der Ester ein t-Butylester ist, durch Behandeln mit einer wasserfreien Säure.

Carbonsäuren der allgemeinen Formel I können mit pharmazeutisch annehmbaren Basen, gemäss Verfahrensvariante (1) des erfindungsgemässen Verfahrens, in Salze überführt werden. Demnach können beispielsweise Carbonsäuren der allgemeinen Formel I durch Behandeln mit Alkalimetall-Hydroxiden, wie Natriumhydroxid und Kaliumhydroxid, Erdalkalimetall-Hydroxiden, wie Kalziumhydroxid und Magnesiumhydroxid, organischen Basen, wie Dicyclohexylamin, basischen Aminosäuren, wie Lysin und Arginin, in Salze überführt werden.

Es sei an dieser Stelle betont, dass die Verbindungen der allgemeinen Formel I in racemischer oder optisch aktiver Form auftreten können und dass die vorliegende Erfindung nicht nur die Racemate, sondern ebenso die optischen Enantiomeren umfasst. Ein Racemat kann gemäss allgemein üblichen Methoden in die optischen Enantiomeren aufgetrennt werden; beispielsweise durch Chromatographie oder durch fraktionierte Kristallisation eines Salzes mit einer optisch aktiven Base, wie α-Methylbenzylamin.

Die als Ausgangsstoffe verwendeten Verbindungen der allgemeinen Fomel II sind neu und können hergestellt werden, indem man zuerst ein Isocyanat der allgemeinen Formel

$$R^{10}\text{-A-N=C=O} \qquad \text{XII}$$

worin A und $R^{10}$ obige Bedeutung besitzen,

mit einem niederen Alkoxycarbonylhydrazid, wie Aethoxycarbonylhydrazid, umsetzt. Dabei arbeitet man zweckmässigerweise in einem inerten organischen Lösungsmittel, beispielsweise in einem aromatischen Kohlenwasserstoff, wie Benzol, Toluol oder dergleichen, bei Raumtemperatur.

Aus der vorhergehenden Reaktion erhält man ein Semicarbazid der allgemeinen Formel

$$R\text{-}A\text{-}NH\text{-}CO\text{-}NH\text{-}NH\text{-}CO_2R^7 \qquad XIII$$

worin R und A obige Bedeutung besitzen, und $R^7$ niederes Alkyl bedeutet,

das anschliessend zu einer Verbindung der allgemeinen Formel

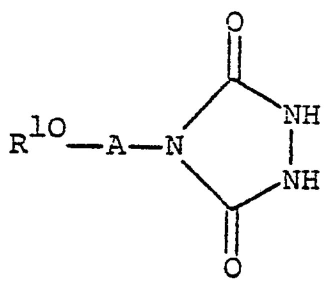

XIV

worin A und $R^{10}$ obige Bedeutung besitzen,

cyclisiert wird.

Die Cyclisierung eines Semicarbazides der allgemeinen Formel XIII zu einer Verbindung der allgemeinen Formel XIV erfolgt durch Behandeln mit einem Alkalimetall-Hydroxid, wie Natriumhydroxid oder, vorzugsweise, Kaliumhydroxid, bei einer Temperatur zwischen etwa 20° und 100°C, vorzugsweise bei 100°C, und anschliessendes Ansäuern, beispielsweise mit einer Mineralsäure, wie Salzsäure, oder mit einem sauren Ionenaustauscher, und, falls erwünscht, durch erneute Veresterung in an sich bekannter Weise, beispielsweise mit methanolischer Salzsäure.

Schliesslich kann eine Verbindung der allgemeinen Formel XIV zu einer Verbindung der allgemeinen Formel II

oxidiert werden. Geeignete Oxidationsmittel umfassen t-Butylhypochlorit, Stickstofftetroxid, Bleitetraacetat und dergleichen. Diese Oxidation wird zweckmässigerweise in einem unter der Verfahrensvariante (a) aufgeführten Lösungsmittel bei einer Temperatur zwischen etwa -10° und +30°C, vorzugsweise bei Raumtemperatur, durchgeführt. Wie bereits früher erwähnt, ist es vorteilhaft eine so erhaltene Verbindung der allgemeinen Formel II nicht zu isolieren, sondern sie, gemäss Verfahrensvariante (a) in Lösung direkt weiter zu verarbeiten.

Die als Ausgangsstoffe verwendeten Verbindungen der allgemeinen Formel III sind entweder bekannt, oder können nach an sich bekannten Verfahren hergestellt werden.

Die als Ausgangsstoffe verwendeten Verbindungen der allgemeinen Formeln IV, V und VII gehören an sich bekannten Stoffklassen an.

Ausgangsstoffe der allgemeinen Formel VI, worin $R^2$ Hydroxy bedeutet, in 6,7-Stellung eine Doppelbindung vorhanden ist, und $R^5$ Wasserstoff bedeutet sind bekannte Verbindungen. Die übrigen Ausgangsverbindungen der allgemeinen Formel VI, worin in 6,7-Stellung eine Doppelbindung vorhanden ist, können in Analogie zu den bekannten Verbindungen hergestellt werden. Ausgangsverbindungen der allgemeinen Formel VI, worin in 6,7-Stellung eine Einfachbindung vorhanden ist, können in Analogie zu den Verfahrensvarianten (e), (h) oder (i) aus Verbindung der allgemeinen Formel VI, worin in 6,7-Stellung eine Doppelbindung vorhanden ist, hergestellt werden.

Die als Ausgangsstoffe verwendeten Verbindungen der allgemeinen Formel VIII sind neu und können hergestellt werden, indem man Verbindungen der allgemeinen Formel VI mit Formaldehyd behandelt. Dabei verwendet man vorzugsweise wässriges Formaldehyd bei erhöhter Temperatur, zweckmässigerweise bei etwa 100°C.

Die Ausgangsverbindungen der allgemeinen Formel XI sind neu und können hergestellt werden, indem man Verbindungen der allgemeinen Formel I, worin in 6,7-Stellung eine Doppelbindung vorhanden ist, mit Quecksilberacetat behandelt. Diese Reaktion wird vorzugsweise in Gegenwart eines Alkalimetallacetats, wie Natriumacetat, in Eisessig, oder in einer Mischung aus Tetrahydrofuran und Wasser oder in einem niederen Alkanol, wie Methanol, durchgeführt. Diese Reaktion wird vorteilhafter Weise bei erhöhter Temperatur durchgeführt, vorzugsweise bei der Siedetemperatur der Reaktionsmischung. Das in dieser Reaktion gebildete Quecksilber kann mittels physikalischer Methoden, wie Dekantieren und dergleichen, oder durch Behandeln mit einem Alkalimetallborhydrid, wie Natriumborhydrid, entfernt werden. Diese Reaktion führt zu einer Mischung von Verbindungen der allgemeinen Formeln

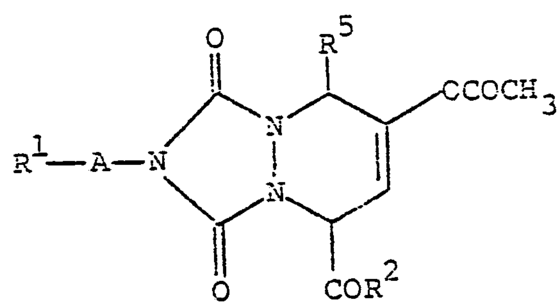

IX

und

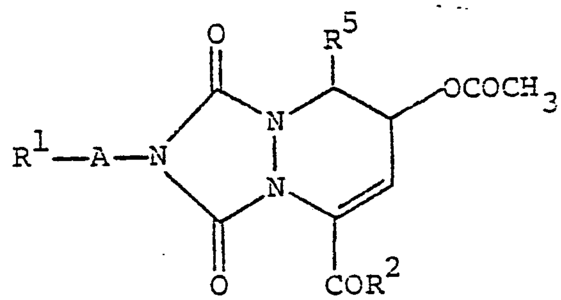

X

worin A, $R^1$, $R^2$ und $R^5$ jeweils obige Bedeutung besitzen.

In der nächsten Stufe wird die Mischung von Verbindungen der allgemeinen Formeln IX und X katalytisch hydriert. Diese katalytische Hydrierung kann in Analogie zur weiter oben beschriebenen Verfahrensvariante (b) durchgeführt werden. Vorzugsweise verwendet man Rhodium auf Aluminium als Katalysator und arbeitet in Eisessig. Durch diese katalytische Hydrierung erhält man Verbindungen der allgemeinen Formel XI.

Verbindungen der allgemeinen Formel II, Verbindungen der allgemeinen Formel VI (mit der Einschränkung, dass $R^5$ nicht Wasserstoff ist, wenn das Molekül in 6,7-Stellung eine Doppelbindung besitzt und $R^2$ Hydroxy bedeuet), Verbindungen der allgemeinen Formel VIII, Verbindungen der allgemeinen Formel XI und Verbindungen der allgemeinen Formel XIV bilden ebenfalls Gegenstand der vorliegenden Erfindung.

Die neuen Triazolopyridazin-Derivate sind nützlich als antihypertensive Wirkstoffe. Sie hemmen das Angiotensin umwandelnde Enzym (ACE), das die Umwandlung von Angiotensin I zu Angiotensin II zustande bringt, und sind deshalb nützlich, um eine durch Angiotensin bewirkte erhöhte Muskelspannung zu vermindern oder zu lindern.

Die Aktivität der vorliegenden Triazolopyridazin-Derivate das das Angiotensin umwandelnde Enzym (ACE) in vitro zu hemmen kann durch den folgenden Test bestimmt werden.

Die verwendete Methode basiert auf einer Methode von Cushman und Cheung (Biochem. Pharmacol. 20, 1637-1648) unter Berücksichtigung der Modifikationen von Hayakari et al. (Anal. Biochem. 84, 361-369). Das Substrat (Hippurylhistidyl-leucin, 2 mM) wird mit oder ohne Testverbindung

(verschiedene Konzentrationen) in Kaliumphosphat-Puffer (pH 8,3; 100 mM), das Natriumchlorid (300 mM) enthält, während 25 Minuten bei 37°C (Gesamtwert 500 µl) mit ACE inkubiert. Die Reaktion wird durch Zugabe von 3 ml Kaliumphosphat-Puffer (pH 8,3; 200 mM) bei 0°C abgebrochen. Man versetzt anschliessend mit 2,4,6-Trichlor-s-triazin (3%) in 1,5 ml Dioxan und schüttelt die erhaltene Mischung, bis der gelbe Chromophor sich voll entwickelt hat. Die Proben werden dann zentrifugiert um allenfalls entstandene Festkörper zu entfernen. Der gelbe Chromophor, der durch Reaktion von 2,4,6-Trichlor-s-triazin mit freier Hippursäure entstanden ist, wird spektrophotometrisch bei 382 nm ausgemessen. Der $IC_{50}$-Wert ist diejenige Konzentration einer Testverbindung, die notwendig ist, um die Spaltung von Hippuryl-histidyl-leucin durch ACE unter den vorangehend beschriebenen Bedingungen um 50% zu reduzieren.

Die in obigem Test erhaltenen Resultate mit repräsentativen Vertretungen von Verbindungen der allgemeinen Formel I sind in der folgenden Tabelle zusammengestellt.

Verbindung A: 5-Carboxy-2,3,5,8-tetrahydro-1,3-dioxo-1H-1,2,4-triazolo[1,2-a]pyridazin-2-propionsäure.

Verbindung B: 2-(2-Carbamoyläthyl)-2,3,5,8-tetrahydro-1,3-dioxo-1H-1,2,4-triazolo[1,2-a]-pyridazin-5-carbonsäure.

Verbindung C: 2-[2-(N-Hydroxycarbamoyl)äthyl]-2,3,5,8-tetrahydro-1,3-dioxo-1H-1,2,4-triazolo-[1,2-a]pyridazin-5-carbonsäure.

Verbindung D: 2,3,5,8-Tetrahydro-2-(2-mercaptoäthyl)-1,3-dioxo-1H-1,2,4-triazolo[1,2-a]-pyridazin-5-carbonsäure.

Tabelle

| Verbindung | $IC_{50}$ (M) |
|---|---|
| A | $1,8 \times 10^{-5}$ |
| B | $6,5 \times 10^{-5}$ |
| C | $3,3 \times 10^{-6}$ |
| D | $4,8 \times 10^{-7}$ |

Die Triazolopyridazin-Derivate der allgemeinen Formel I können in Form von pharmazeutischen Präparaten, welche Verbindungen der allgemeinen Formel I zusammen mit geeigneten pharmazeutischen Trägermaterialien enthalten, als Medikamente verwendet werden. Als Trägermaterialien kommen dabei für die enterale, beispielsweise orale, oder parenterale Verabreichung geeignete organische oder inorganische Trägermaterialien in Frage, beispielsweise Wasser, Gelatine, Gummi arabicum, Lactose, Stärke, Magnesiumstearat, Talk, pflanzliche Oele, Polyalkylenglycole, Vaselin und dergleichen. Die pharmazeutischen Präparate können in fester Form, beispielsweise als Tabletten, Dragées, Suppositorien oder Kapseln, oder in flüssiger Form, beispielsweise als Lösungen, Suspensionen oder Emulsionen, verarbeitet werden. Die pharmazeutischen Präparate können üblichen pharmazeutischen Behandlungen, wie Sterilisation, unterworfen werden, und/oder Zusatzstoffe enthalten, wie Konservierungsmittel, Stabilisatoren, Netzmittel oder Emulgatoren, Salze, um den osmotischen Druck zu variieren, oder Puffer. Die pharmazeutischen Präparate können aber auch andere therapeutisch wertvolle Verbindungen enthalten.

Die Triazolopyridazin-Derivate, gemäss vorliegender Erfindung, können Erwachsenen in einer täglichen Dosis von etwa 0,1 bis 100 mg, vorzugsweise ca. 1 bis 50 mg, pro kg Körpergewicht verabreicht werden. Die tägliche Dosis kann

in einer Dosis oder in verschiedenen Dosen verabreicht werden. Es sei an dieser Stelle betont, dass die oben angegebenen Dosierungen als Beispiele angegeben wurden, und dass sie, je nach Stärke der behandelten Symptome und Zustand der Patienten, nach oben oder nach unten variieren können und letztlich vom behandelnden Arzt festgelegt werden.

Die nachfolgenden Beispiele dienen der näheren Erläuterung der vorliegenden Erfindung.

## Beispiel 1

A) Eine Lösung von 18,0 g (0,17 Mol) Aethoxycarbonylhydrazin in 400 ml trockenem Toluol wird unter Rühren tropfenweise mit einer Lösung von 19,72 g (0,17 Mol) Methoxycarbonylmethyl-isocyanat in 70 ml trockenem Toluol versetzt. Nach Rühren über Nacht bei Raumtemperatur wird der ausgefallene Festkörper abfiltriert, mit trockenem Toluol gewaschen, getrocknet und aus Essigester umkristallisiert. Man erhält 28,54 g (76%) 1-Aethoxycarbonyl-4-methoxycarbonylmethyl-semicarbazid vom Schmelzpunkt 116-121°C.

B) Eine Lösung von 27,47 g (0,125 Mol) 1-Aethoxycarbonyl-4-methoxycarbonylmethyl-semicarbazid in 95 ml (0,38 Mol) 4M Kaliumhydroxid wird während 1 1/2 Stunden auf dem Dampfbad erwärmt, anschliessend auf Raumtemperatur abgekühlt, mit konzentrierter Salzsäure auf pH 1 gestellt und im Vakuum zu einem farblosen Festkörper eingedampft. Dieser Festkörper wird 3 x mit je 90 ml siedendem Methanol extrahiert. Die Auszüge werden filtriert, vereinigt und zu 400 ml methanolischer Salzsäure gegeben. Nach Stehen über Nacht bei Raumtemperatur wird die Mischung im Vakuum eingedampft. Der farblose Rückstand wird aus Methanol umkristallisiert und ergibt 7,5 g (35%) Methyl-3,5-dioxo-1,2,4-triazolidin-4-acetat vom Schmelzpunkt 215-218°C.

C) Eine Mischung aus 0,5 g (2,9 mMol) Methyl-3,5-dioxo-1,2,4-triazolidin-4-acetat und 8,7 ml (8,7 mMol) 1N Natronlauge wird während 2 Stunden bei Raumtemperatur gerührt. Die erhaltene Lösung wird mit konzentrierter Salzsäure sauer gestellt und zur Trockene eingedampft. Der feste Rückstand wird mit siedendem Acetonitril extrahiert. Nach Eindampfen der Acetonitril-Auszüge wird der farblose Rückstand aus Acetonitril umkristallisiert. Man erhält dabei 0,15 g (33%) 3,5-Dioxo-1,2,4-triazolidin-4-essigsäure vom Schmelzpunkt 220-223°C.

D) Eine Suspension von 1,69 g (0,011 Mol) 3,5-Dioxo-1,2,4-triazolidin-4-essigsäure in 20 ml trockenem Dioxan wird unter einer Stickstoffatmosphäre bei Raumtemperatur gerührt. Ueber einen Zeitraum von 15 Minuten gibt man tropfenweise eine Lösung von 1,32 g (0,012 Mol) t-Butylhypochlorit in 4 ml trockenem Dioxan hinzu. Nach 45 Minuten wird die erhaltene, rot gefärbte Lösung filtriert und im Vakuum zu einem roten Festkörper eingedampft. Der Rückstand wird in 30 ml Dioxan aufgenommen und portionenweise zu einer gerührten Lösung von 1,25 g (0,013 Mol) Penta-2,4-diencarbonsäure in 20 ml Dioxan gegeben. Zwischen den einzelnen Zugaben wartet man bis die rote Farbe verblasst ist. Nach Beengigung der Zugabe und Erblassen der roten Färbung wird die Lösung noch während einer Stunde bei Raumtemperatur gerührt und anschliessend im Vakuum zu einem farblosen Festkörper eingedampft. Dieser Festkörper wird aus Acetonitril umkristallisiert und ergibt 1,17 g (43%) 5-Carboxy-2,3,5,8-tetrahydro-1,3-dioxo-1H-1,2,4-triazolo-[1,2-a]pyridazin-2-essigsäure vom Schmelzpunkt 209-211°C (Zersetzung).

## Beispiel 2

A) Aus 2-Methoxycarbonyläthyl-isocyanat erhält man in Analogie zu Beispiel 1 A) in 70%-iger Ausbeute 1-Aethoxycarbonyl-4-(2-methoxycarbonyläthyl)-semicarbazid vom Schmelzpunkt 105-107°C.

B) Aus 1-Aethoxycarbonyl-4-(2-methoxycarbonyläthyl)-semicarbazid erhält man in Analogie zu Beispiel 1 B) in 42%-iger Ausbeute Methyl-3,5-dioxo-1,2,4-triazolidin-4-propionat vom Schmelzpunkt 150-153°C.

C) Aus Methyl-3,5-dioxo-1,2,4-triazolidin-4-propionat erhält man in Analogie zu Beispiel 1 C) 3,5-Dioxo-1,2,4-triazolidin-4-propionsäure vom Schmelzpunkt 180-183°C (aus Acetonitril).

D) Aus 3,5-Dioxo-1,2,4-triazolidin-4-propionsäure erhält man in Analogie zu Beispiel 1 D) in 63%-iger Ausbeute 5-Carboxy-2,3,5,8-tetrahydro-1,3-dioxo-1H-1,2,4-triazolo [1,2-a]pyridazin-2-propionsäure vom Schmelzpunkt 172-175°C (aus Acetonitril).

Beispiel 3

A) Aus 3-Methoxycarbonylpropyl-isocyanat erhält man in Analogie zu Beispiel 1 A) in 76%-iger Ausbeute 1-Aethoxycarbonyl-4-(3-methoxycarbonylpropyl)-semicarbazid vom Schmelzpunkt 95-96°C.

B) Eine Lösung von 494 mg (2 mMol) 1-Aethoxycarbonyl-4-(3-methoxycarbonylpropyl)-semicarbazid in 1,5 ml (6 mMol) 4N Kalilauge wird während einer Stunde auf dem Dampfbad erwärmt. Die erhaltene Lösung wird auf eine Ionenaustauscher-Säule gegeben (Dowex 50X-8,$H^+$ Form) und mit Wasser eluiert. Nach Eindampfen des Eluates im Vakuum wird der farblose Festkörper aus Acetonitril umkristallisiert. Man erhält 120 mg (32%) 3,5-Dioxo-1,2,4-triazolidin-4-buttersäure vom Schmelzpunkt 168-170°C.

C) Aus 3,5-Dioxo-1,2,4-triazolidin-4-buttersäure erhält man in Analogie zu Beispiel 1 D) in 44%-iger Ausbeute 5-Carboxy-2,3,5,8-tetrahydro-1,3-dioxo-1H-1,2,4-triazolo [1,2-a]pyridazin-2-buttersäure vom Schmelzpunkt 130°C (aus Acetonitril).

Beispiel 4

A) Aus 2-Chloräthyl-isocyanat erhält man in Analogie zu Beispiel 1 A) in 73%-iger Ausbeute 4-(2-Chloräthyl)-1-äthoxycarbonyl-semicarbazid vom Schmelzpunkt 118-120°C.

B) Eine Mischung aus 23,9 g (0,004 Mol) 4-(2-Chloräthyl)-1-äthoxycarbonyl-semicarbazid und 57 ml 0,228 Mol) 4N

Kalilauge wird während 2 Stunden bei Raumtemperatur gerührt. Die Mischung wird mit 100 ml Wasser verdünnt und auf eine Ionenaustauscher-Säule (Dowex 50X-8, $H^+$ Form) gegeben. Nach Eluieren mit Wasser dampft man im Vakuum ein und kristallisiert den erhaltenen farblosen Festkörper aus Wasser um. Man erhält 7,97 g (43%) 4-(2-Chloräthyl)-1,2,4-triazolidin-3,5-dion vom Schmelzpunkt 192-193°C.

C) Aus 4-(2-Chloräthyl)-1,2,4-triazolidin-3,5-dion erhält man in Analogie zu Beispiel 1 D) in 85%-iger Ausbeute 2-(2-Chloräthyl)-2,3,5,8-tetrahydro-1,3-dioxo-1H-1,2,4-triazolo[1,2-a]pyridazin-5-carbonsäure vom Schmelzpunkt 150-151°C.

## Beispiel 5

A) Aus 3-Chlorpropylisocyanat erhält man in Analogie zu Beispiel 1 A) in 78%-iger Ausbeute 4-(3-Chlorpropyl)-1-äthoxycarbonyl-semicarbazid vom Schmelzpunkt 105-106°C.

B) Aus 4-(3-Chlorpropyl)-1-äthoxycarbonyl-semicarbazid erhält man in Analogie zu Beispiel 4 B) in 14%-iger Ausbeute 4-(3-Chlorpropyl)-1,2,4-triazolidin-3,5-dion vom Schmelzpunkt 168-170°C.

C) Aus 4-(3-Chlorpropyl)-1,2,4-triazolidin-3,5-dion erhält man in Analogie zu Beispiel 1 D) 2-(3-Chlorpropyl)-2,3,5,8-tetrahydro-1,3-dioxo-1H-1,2,4-triazolo[1,2-a]pyridazin-5-carbonsäure.

## Beispiel 6

Aus Methyl-3,5-dioxo-1,2,4-triazolidin-4-propionat erhält man in Analogie zu Beispiel 1 D) in 50%-iger Ausbeute Methyl-5-carboxy-2,3,5,8-tetrahydro-1,3-dioxo-1H-1,2,4-triazolo[1,2-a]pyridazin-2-propionat vom Schmelzpunkt 150-153°C (aus Acetonitril).

Racemisches Methyl-5-carboxy-2,3,5,8-tetrahydro-1,3-dioxo-1H-1,2,4-triazolo[1,2-a]pyridazin-2-propionat kann über das entsprechende α-Methylbenzylammoniumsalz in die optischen Antipoden aufgetrennt werden. Man erhält das (+)-Enantiomer vom Schmelzpunkt 180-181°C /$[\alpha]_{365}^{20}$ = +1184° (c = 0,5 in Methanol)/ und das (-)-Enantiomer vom Schmelzpunkt 180-181°C /$[\alpha]_{365}^{20}$ = -1181° (c = 0,5 in Methanol)/.

## Beispiel 7

Aus 3,5-Dioxo-1,2,4-triazolidin-4-propionsäure und Hexa-2,4-diencarbonsäure erhält man in Analogie zu Beispiel 1 D) in 76%-iger Ausbeute 5-Carboxy-2,3,5,8-tetrahydro-8-methyl-1,3-dioxo-1H-1,2,4-triazolo[1,2-a]pyridazin-2-propionsäure vom Schmelzpunkt 184-186°C (aus Acetonitril).

## Beispiel 8

Aus 3,5-Dioxo-1,2,4-triazolidin-4-propionsäure und 5-Phenylpenta-2,4-diencarbonsäure erhält man in Analogie zu Beispiel 1 D) in 23%-iger Ausbeute 5-Carboxy-2,3,5,8-tetrahydro-8-phenyl-1,3-dioxo-1H-1,2,4-triazolo[1,2-a]pyridazin-2-propionsäure vom Schmelzpunkt 177-178°C.(aus Acetonitril).

## Beispiel 9

Eine Lösung von 1,3 g (0,005 Mol) 2-(2-Chloräthyl)-2,3,5,8-tetrahydro-1,3-dioxo-1H-1,2,4-triazolo[1,2-a]pyridazin-5-carbonsäure in 10 ml (0,10 Mol) 1N Natronlauge wird mit 1 ml (0,008 Mol) Benzylmercaptan behandelt und während 3 Stunden bei 100°C gerührt. Die Mischung wird mit konzentrierter Salzsäure angesäuert und 2 x mit je 20 ml Essigester extrahiert. Der organische Auszug wird mit 50 ml gesättigter Natriumbicarbonat-Lösung extrahiert. Der wässrige Auszug wird mit konzentrierter Salzsäure sauer gestellt und 2 x mit je 25 ml Essigester zurückextrahiert. Die organischen Auszüge werden über Magnesiumsulfat getrocknet und eingedampft. Man erhält 2-(2-Benzylthioäthyl)-2,3,5,8-tetrahydro-1,3-dioxo-1H-1,2,4-triazolo[1,2-a]pyridazin-5-carbonsäure als farbloses Oel.

Dieses Oel wird in 10 ml Acetonitril gelöst und mit 2 ml Dicyclohexylamin behandelt. Der ausgefallene farblose Festkörper wird abfiltriert und aus Acetonitril umkristallisiert. Es resultiert 1,83 g (69%) 2-(2-Benzylthioäthyl)-2,3,5,8-tetrahydro-1,3-dioxo-1H-1,2,4-triazolo[1,2-a]pyridazin-5-carbonsäure-dicyclohexylammoniumsalz vom Schmelzpunkt 201-203°C.

## Beispiel 10

Eine Mischung aus 3,24 g (0,0125 Mol) Methyl-2-chlormethyl-2,3,5,8-tetrahydro-1,3-dioxo-1H-1,2,4-triazolo[1,2-a]pyridazin-5-carboxylat, 2,29 g (0,02 Mol) Kaliumthioacetat in 400 ml Aceton wird zusammen mit einer katalytischen Menge Natriumjodid während 19 Stunden unter Rückfluss zum Sieden erhitzt. Nach Entfernen des Acetons wird der Rückstand zwischen 250 ml Essigester und 200 ml gesättigter Natriumchlorid-Lösung verteilt. Die organische Phase wird abgetrennt, über Magnesiumsulfat getrocknet und eingedampft. Das erhaltene gelbe Oel wird unter Eluieren mit Chloroform/Essigester (1:1) an Kieselgel chromatographiert. Man erhält 2,19 g (59%) Methyl-2-acetylthiomethyl-2,3,5,8-tetrahydro-1,2-dioxo-1H-1,2,4-triazolo[1,2-a]pyridazin-5-carboxylat als farbloser Festkörper vom Schmelzpunkt 110-111°C (aus Chloroform/Hexan).

## Beispiel 11

Aus t-Butyl-2-(2-chloräthyl)-2,3,5,8-tetrahydro-1,3-dioxo-1H-1,2,4-triazolo[1,2-a]pyridazin-5-carboxylat und Kaliumthioacetat erhält man in Analogie zu Beispiel 10 t-Butyl-2-(2-acetyl-thioäthyl)-2,3,5,8-tetrahydro-1,3-dioxo-1H-1,2,4-triazolo[1,2-a]pyridazin-5-carboxylat vom Schmelzpunkt 102-103°C.

Beispiel 12

Eine Lösung von 1,75 g (0,005 Mol) t-Butyl-2-(2-acetylthioäthyl)-2,3,5,8-tetrahydro-1,3-dioxo-1H-1,2,4-triazolo[1,2-a]pyridazin-5-carboxylat in 20 ml Trifluoressigsäure wird während einer Stunde stehengelassen und anschliessend eingedampft. Man erhält 2-(2-Acetylthioäthyl)-2,3,5,8-tetrahydro-1,3-dioxo-1H-1,2,4-triazolo[1,2-a]pyridazin-5-carboxylat. Eine Lösung dieser Carbonsäure in 60 ml Wasser wird unter Stickstoff mit 0,88 ml Ammoniak behandelt. Die Mischung wird während einer Stunde bei Raumtemperatur gerührt, mit konzentrierter Salzsäure sauer gestellt, mit Natriumchlorid gesättigt und 3 x mit je 150 ml Chloroform ausgeschüttelt. Die Chloroform-Auszüge werden über Magnesiumsulfat getrocknet und zu einem farblosen Festkörper eingedampft. Dieser Festkörper kristallisiert aus Essigester/Hexan und ergibt 0,92 g (73%) 2,3,5,8-Tetrahydro-2-(2-mercaptoäthyl)-1,3-dioxo-1H-1,2,4-triazolo[1,2-a]pyridazin-5-carbonsäure vom Schmelzpunkt 156-158°C.

Beispiel 13

Eine Lösung von 5 g (0,0147 Mol) Methyl-2,3,5,8-tetrahydro-5-t-butyloxycarbonyl-1,3-dioxo-1H-1,2,4-triazolo[1,2-a]pyridazin-2-propionat in 30 ml Methanol wird mit 8,24 g (0,118 Mol) Hydroxylamin-hydrochlorid und 6,41 g (0,118 Mol) Kaliumhydroxid behandelt. Die erhaltene Suspension wird über Nacht bei Raumtemperatur gerührt, mit 4,12 g (0,059 Mol) Hydroxylamin-hydrochlorid und 3,20 g (0,059 Mol) Kaliumhydroxid versetzt und während weiteren 88 Stunden bei Raumtemperatur gerührt. Nach Eindampfen der Mischung zur Trockene und Aufnehmen des Rückstandes in

80 ml Wasser wird die Lösung mit 4N Kalilauge basisch gestellt und 2 x mit je 150 ml Aether ausgeschüttelt. Die wässrige Phase wird mit 2N Schwefelsäure sauer gestellt, mit Natriumchlorid gesättigt und 4 x mit je 100 ml Chloroform ausgeschüttelt. Die Chloroform-Auszüge werden über Magnesiumsulfat getrocknet und eingedampft. Das erhaltene gelbe Oel kristallisiert aus 100 ml Aether, das einige Tropfen Wasser enthält, und liefert 3,81 g (76%) t-Butyl-2-[2-(N-hydroxycarbamoyl)äthyl]-2,3,5,8-tetrahydro-1,3-dioxo-1H-1,2,4-triazolo[1,2-a]pyridazin-5-carboxylat vom Schmelzpunkt 75-78°C.

Der obige Ester wird mit 35%-igem Bromwasserstoff in Essigsäure gelöst und während einer Stunde bei Raumtemperatur stehen gelassen. Durch Zugabe von Aether wird das Produkt ausgefällt und anschliessend gefriergetrocknet. Dabei erhält man in 80%-iger Ausbeute 2-[2-(N-Hydroxycarbamoyl)-äthyl]-2,3,5,8-tetrahydro-1,3-dioxo-1H-1,2,4-triazolo[1,2-a]pyridazin-5-carboxylat.

## Beispiel 14

Eine Lösung von 2,83 g (0,01 Mol) Methyl-5-carboxy-2,3,5,8-tetrahydro-1,3-dioxo-1H-1,2,4-triazolo[1,2-a]pyridazin-2-propionat in 50 ml Methanol wird mit Ammoniak gesättigt und während 72 Stunden gerührt. Die Mischung wird anschliessend zur Trockene eingedampft, und der erhaltene Rückstand in Wasser aufgenommen. Die wässrige Lösung wird über eine Ionenaustauscher-Kolonne (Zerolit 225, $H^+$ Form) gelassen und das Eluat zu einem farblosen Festkörper eingedampft. Durch Umkristallisieren aus Methanol erhält man 2,26 g (85%) 2-(2-Carbamoyläthyl)-2,3,5,8-tetrahydro-1,3-dioxo-1H-1,2,4-triazolo[1,2-a]pyridazin-5-carbonsäure vom Schmelzpunkt 218-220°C (Zersetzung).

Aus Methyl-5-methoxycarbonyl-2,3,5,8-tetrahydro-1,3-dioxo-1H-1,2,4-triazolo[1,2-a]pyridazin-2-propionat und

methanolischem Ammoniak erhält man in analoger Weise in 50%-iger Ausbeute 5-Carbamoyl-2,3,5,8-tetrahydro-1,3-dioxo-1H-1,2,4-triazolo[1,2-a]pyridazin-2-propionamid-monohydrat vom Schmelzpunkt 240-243°C (gefriergetrocknet).

## Beispiel 15

Eine Lösung von 283 mg (1 mMol) Methyl-5-carboxy-2,3,5,8-tetrahydro-1,3-dioxo-1H-1,2,4-triazolo[1,2-a]pyridazin-2-propionat in 5 ml Methanol wird mit 1,5 ml (25 mMol) 85%-igem Hydrazinhydrat behandelt. Die Mischung wird während 24 Stunden bei Raumtemperatur gerührt. Der farblose Niederschlag wird abfiltriert und aus denaturiertem Alkohol umkristallisiert. Man erhält 50 mg (18%) 2-[(2-(Hydrazinocarbonyl)äthyl]-2,3,5,8-tetrahydro-1,3-dioxo-1H-1,2,4-triazolo[1,2-a]pyridazin-5-carbonsäure vom Schmelzpunkt 165-170°C (Zersetzung).

## Beispiel 16

Eine Lösung von 0,75 g 5-Carboxy-2,3,5,8-tetrahydro-1,3-dioxo-1H-1,2,4-triazolo[1,2-a]pyridazin-2-essigsäure in 50 ml Dioxan wird in der Gegenwart von 10% Palladium auf Kohle während 3 Stunden bei Raumtemperatur hydriert. Nach Abfiltrieren vom Katalysator wird zu einem farblosen Festkörper eingedampft. Nach Umkristallisieren aus Acetonitril resultieren 0,50 g (67%) 5-Carboxy-2,3,5,6,7,8-hexahydro-1,3-dioxo-1H-1,2,4-triazolo[1,2-a]pyridazin-2-essigsäure vom Schmelzpunkt 224-226°C (aus Acetonitril).

Die folgenden Verbindungen wurden in analoger Weise hergestellt:

5-Carboxy-2,3,5,6,7,8-hexahydro-1,3-dioxo-1H-1,2,4-triazolo[1,2-a]pyridazin-2-propionsäure vom Schmelzpunkt 189-192°C,

5-Carboxy-2,3,5,6,7,8-hexahydro-1,3-dioxo-1H-1,2,4-triazolo[1,2-a]pyridazin-2-buttersäure vom Schmelzpunkt 147-148°C,

Methyl-2,3,5,6,7,8-hexahydro-1,3-dioxo-1H-1,2,4-triazolo[1,2-a]pyridazin-2-propionat vom Schmelzpunkt 147-148°C, und

2-(2-Chloräthyl)-2,3,5,6,7,8-hexahydro-1,3-dioxo-1H-1,2,4-triazolo[1,2-a]pyridazin-5-carbonsäure vom Schmelzpunkt 151-152°C.

## Beispiel 17

Eine Mischung aus 7,25 g (0,036 Mol) Hexahydro-1,3-dioxo-1H-1,2,4-triazolo[1,2-a]pyridazin-5-carbonsäure und 250 ml methanolischer Salzsäure wird während 2 Stunden unter Rückfluss zum Sieden erhitzt. Nach Entfernen des Lösungsmittels wird der Rückstand aus Methanol umkristallisiert und ergibt 5,05 g (65%) Methyl-hexahydro-1,3-dioxo-1H-1,2,4-triazolo[1,2-a]pyridazin-5-carboxylat vom Schmelzpunkt 215-216°C.

Eine Lösung von 1,50 g (0,007 Mol) Methyl-hexahydro-1,3-dioxo-1H-1,2,4-triazolo[1,2-a]pyridazin-5-carboxylat in 40 ml trockenem t-Butanol wird mit einer Lösung von 0,87 g (0,008 Mol) Kalium-t-Butoxid in 50 ml trockenem t-Butanol versetzt und unter einer Stickstoffatmosphäre während 4 Stunden unter Rückfluss zum Sieden erhitzt. Nach Zugabe von 1,61 g (0,007 Mol) Benzylbromacetat wird während weiteren 4 Stunden erhitzt. Die Mischung wird anschliessend filtriert und eingedampft. Den Rückstand chromatographiert man an Kieselgel unter Eluieren mit Chloroform. Man erhält 1,32 g (52%) Benzyl-5-methoxycarbonyl-2,3,5,6,7,8-hexahydro-1,3-dioxo-1H-1,2,4-triazolo[1,2-a]pyridazin-2-acetat vom Schmelzpunkt 103-106°C (aus Methylenchlorid/Hexan).

Alkylieren von Methyl-hexahydro-1,3-dioxo-1H-1,2,4-triazolo[1,2-a]pyridazin-5-carboxylat mit 1-Brom-2-

chloräthan in analoger Weise wie oben ergibt Methyl-2-(2-chloräthyl)-2,3,5,6,7,8-hexahydro-1,3-dioxo-1H-1,2,4-triazolo[1,2-a]pyridazin-5-carboxylat vom Schmelzpunkt 94-95°C.

Beispiel 18

Aus 2,3,5,8-Tetrahydro-1,3-dioxo-1H-1,2,4-triazolo[1,2-a]pyridazin-5-carbonsäure und methanolischer Salzsäure erhält man in Analogie zu Absatz 1 von Beispiel 17 in 74%-iger Ausbeute Methyl-2,3,5,8-tetrahydro-1,3-dioxo-1H-1,2,4-triazolo[1,2-a]pyridazin-5-carboxylat vom Schmelzpunkt 210-213°C (aus Methanol).

Eine Mischung aus 7,02 g (0,033 Mol) Methyl-2,3,5,8-tetrahydro-1,3-dioxo-1H-1,2,4-triazolo[1,2-a]pyridazin-5-carboxylat und 9 ml 40%-igem Formalin wird auf dem Dampfbad während 4 1/2 Stunden erhitzt. Nach Eindampfen der Mischung im Vakuum wird das erhaltene farblose Oel aus Methylenchlorid/Hexan kristallisiert. Man erhält 3,97 g (50%) Methyl-2-hydroxymethyl-2,3,5,8-tetrahydro-1,3-dioxo-1H-1,2,4-triazolo[1,2-a]pyridazin-5-carboxylat vom Schmelzpunkt 123-125°C.

Methyl-2-hydroxymethyl-2,3,5,8-tetrahydro-1,3-dioxo-1H-1,2,4-triazolo[1,2-a]pyridazin-5-carboxylat kann auch auf folgendem Weg hergestellt werden:

2,3,5,8-Tetrahydro-1,3-dioxo-1H-1,2,4-triazolo[1,2-a]pyridazin-5-carbonsäure wird wie oben beschrieben mit Formaldehyd umgesetzt. Man erhält in 44%-iger Ausbeute 2,3,5,8-Tetrahydro-2-hydroxymethyl-1,3-dioxo-1H-1,2,4-triazolo[1,2-a]pyridazin-5-carbonsäure vom Schmelzpunkt 175-178°C (aus Isopropanol). Nach Veresterung dieser Säure mit Diazomethan in Aether erhält man Methyl-2-hydroxymethyl-2,3,5,8-tetrahydro-1,3-dioxo-1H-1,2,4-triazolo[1,2-a]pyridazin-5-carboxylat vom Schmelzpunkt 123-125°C.

Eine Suspension von 3,97 g (0,017 Mol) Methyl-2-hydroxymethyl-2,3,5,8-tetrahydro-1,3-dioxo-1H-1,2,4-triazolo[1,2-a]pyridazin-5-carboxylat in 300 ml trockenem Aether wird bei 0°C portionenweise mit 3,49 g (0,017 Mol) Phosphorpentachlorid versetzt und während 22 Stunden bei Raumtemperatur gerührt. Nach Eindampfen der erhaltenen klaren Lösung im Vakkum wird der Rückstand an Kieselgel unter Eluieren mit Chloroform/Essigester (1:1) chromatographiert. Man erhält 3,24 g (76%) Methyl-2-chlormethyl-2,3,5,8-tetrahydro-1,3-dioxo-1H-1,2,4-triazolo[1,2-a]pyridazin-5-carboxylat vom Schmelzpunkt 164-165°C.

## Beispiel 19

Bei 0°C versetzt man eine Lösung von 6,19 g (0,023 Mol) 5-Carboxy-2,3,5,8-tetrahydro-1,3-dioxo-1H-1,2,4-triazolo[1,2-a]pyridazin-2-propionsäure in 115 ml 40%-igem wässrigem Bromwasserstoff tropfenweise mit 23 ml (ca. 0,23 Mol) 30%-igem Wasserstoffperoxid. Man rührt während 2 Stunden bei ca. 5°C und dampft anschliessend zur Trockene ein. Der Rückstand wird aus Aceton/Aether kristallisiert und ergibt 3,63 g (37%) 6,7-Dibrom-5-carboxy-hexahydro-1,3-dioxo-1H-1,2,4-triazolo[1,2-a]pyridazin-2-propionsäure vom Schmelzpunkt 221-222°C.

## Beispiel 20

7,14 g (0,024 Mol) Methyl-5-methoxycarbonyl-2,3,5,8-tetrahydro-1,3-dioxo-1H-1,2,4-triazolo[1,2-a]pyridazin-2-propionat werden mit 7,63 g (0,024 Mol) Quecksilberacetat und 3,94 g (0,048 Mol) wasserfreiem Natriumacetat in 60 ml Eisessig während 5 Stunden bei 100°C behandelt. Die erhaltene Lösung wird vom ausgefallenen Quecksilber abdekantiert und zur Trockene eingedampft. Der verbliebene Rückstand wird mehrmals mit Methylenchlorid extrahiert. Nach Eindampfen der Methylenchlorid-Extrakte erhält man 7,8 g (91%) einer Mischung aus Methyl-7-acetoxy-2,3,5,8-tetra-

hydro-5-methoxycarbonyl-1,3-dioxo-1H-1,2,4-triazolo[1,2-a] pyridazin-2-propionat und Methyl-7-acetoxy2,3,7,8-tetrahydro-5-methoxycarbonyl-1,3-dioxo-1H-1,2,4-triazolo[1,2-a] pyridazin-2-propionat als hellgelbes Oel.

2,8 g (0,0078 Mol) der obigen Mischung werden in 20 ml Eisessig gelöst und über 400 mg Rhodium auf Aluminium hydriert. Nach üblichem Aufarbeiten und Chromatographieren an Kieselgel unter Eluieren mit Chloroform/Methanol (95:5) erhält man 1,35 g (48%) Methyl-7-acetoxyhexahydro-5-methoxycarbonyl-1,3-dioxo-1H-1,2,4-triazolo[1,2-a]pyridazin-2-propionat als gelbes Oel.

Dieser Stoff wird in 20 ml 6N Salzsäure während einer Stunde bei 100°C hydrolysiert. Nach Eindampfen und Kristallisieren des Rückstandes aus Aceton erhält man 0,82 g (69%) 5-Carboxy-hexahydro-7-hydroxy-1,3-dioxo-1H-1,2,4-triazolo[1,2-a]pyridazin-2-propionsäure vom Schmelzpunkt 182-183°C.

## Beispiel 21

A) Eine Lösung von 0,1 Mol 2-(2-Chloräthyl)-2,3,5,8-tetrahydro-1,3-dioxo-1H-1,2,4-triazolo[1,2-a]pyridazin-5-carbonsäure in 500 ml Dioxan oder Methylenchlorid wird mit 12 ml konzentrierter Schwefelsäure versetzt und anschliessend mit 500 ml Isobuten behandelt. Man rührt während 4 Tagen bei Raumtemperatur, verdünnt die Mischung mit Wasser und schüttelt mit Aether aus. Die ätherischen Auszüge werden mit 10%-iger Natriumcarbonat-Lösung gewaschen, über Magnesiumsulfat getrocknet und eingedampft. Man erhält t-Butyl-2-(2-chloräthyl)-2,3,5,8-tetrahydro-1,3-dioxo-1H-1,2,4-triazolo[1,2-a]pyridazin-5-carboxylat vom Schmelzpunkt 87-88°C.

B) Eine Suspension von 5 mMol 2-(2-Chloräthyl)-2,3,5,8-tetrahydro-1,3-dioxo-1H-1,2,4-triazolo[1,2-a]pyridazin-5-carbonsäure in 40 ml trockenem Aether wird zusammen mit 1,18 g (5,5 mMol) Phosphorpentachlorid während 3 Stunden bei Raumtemperatur gerührt. Nach Eindampfen der Mischung erhält man das gewünschte Säurechlorid, das in 35 ml t-Butanol aufgenommen und mit 2 ml (15 mMol) N,N-Dimethylanilin behandelt wird. Man rührt die Mischung bei Raumtemperatur über Nacht und dampft anschliessend ein. Nach Verteilen des Rückstandes zwischen Essigester und 2N Salzsäure wird die Essigester-Phase abgetrennt, mit gesättigter Natriumchlorid-Lösung und anschliessend mit gesättigter Natriumbicarbonat-Lösung gewaschen, über Magnesiumsulphat getrocknet und eingedampft. Man erhält t-Butyl-2-(2-chloräthyl)-2,3,5,8-tetrahydro-1,3-dioxo-1H-1,2,4-triazolo[1,2-a]pyridazin-5-carboxylat vom Schmelzpunkt 87-88°C.

Aus Methyl-5-carboxy-2,3,5,8-tetrahydro-1,3-dioxo-1H-1,2,4-triazolo[1,2-a]pyridazin-2-propionat erhält man in Analogie zu vorstehendem Absatz Methyl-2,3,5,8-tetrahydro-5-t-butoxycarbonyl-1,3-dioxo-1H-1,2,4-triazolo[1,2-a]pyridazin-2-propionat vom Schmelzpunkt 77-78°C.

C) Aus 5-Carboxy-2,3,5,8-tetrahydro-1,3-dioxo-1H-1,2,4-triazolo[1,2-a]pyridazin-2-propionsäure erhält man in Analogie zum ersten Absatz von Beispiel 17 Methyl-2,3,5,8-tetrahydro-5-methoxycarbonyl-1,3-dioxo-1H-1,2,4-triazolo [1,2-a]pyridazin-2-propionat vom Schmelzpunkt 91-92°C.

D) Eine Suspension von 5-Carboxy-2,3,5,8-tetrahydro-1,3-dioxo-1H-1,2,4-triazolo[1,2-a]pyridazin-2-propionsäure in trockenem Aether wird mit einem kleinen Ueberschuss ätherischer Diazomethan-Lösung behandelt. Nach 2 Stunden bei Raumtemperatur wäscht man mit 10%-iger Natriumcarbonat-Lösung, trocknet über Magnesiumsulfat und dampft ein. Der Rückstand liefert nach Umkristallisieren aus Methanol Methyl-2,3,5,8-tetrahydro-5-methoxycarbonyl-1,3-dioxo-1H-1,2,4-triazolo[1,2-a]pyridazin-2-propionat vom Schmelzpunkt

91-92°C.

Beispiel 22

Eine Lösung von Methyl-5-carboxy-2,3,5,8-tetrahydro-1,3-dioxo-1H-1,2,4-triazolo[1,2-a]pyridazin-2-propionat in Methanol wird mit 1N Natronlauge (1,1 Aequivalente) während 2 Stunden bei Raumtemperatur gerührt. Nach Ansäuern mit konzentrierter Salzsäure wird die Mischung zur Trockene eingedampft. Der Rückstand wird mit Acetonitril extrahiert. Nach Eindampfen der Auszüge erhält man 5-Carboxy-2,3,5,8-tetrahydro-1,3-dioxo-1H-1,2,4-triazolo[1,2-a]pyridazin-2-propionsäure vom Schmelzpunkt 172-175°C (aus Acetonitril).

Die folgenden Carbonsäuren wurden in analoger Weise durch Verseifung der entsprechenden Ester hergestellt:

(+)-5-Carboxy-2,3,5,8-tetrahydro-1,3-dioxo-1H-1,2,4-triazolo[1,2-a]pyridazin-2-propionsäure vom Schmelzpunkt 172-175°C /$[\alpha]_{365}^{20}$ = +1243° (c = 0,5 in Methanol)/,

(-)-5-Carboxy-2,3,5,8-tetrahydro-1,3-dioxo-1H-1,2,4-triazolo[1,2-a]pyridazin-2-propionsäure vom Schmelzpunkt 172-175°C /$[\alpha]_{365}^{20}$ = -1245° (c = 0,5 in Methanol)/,

2-(2-Chloräthyl)-2,3,5,6,7,8-hexahydro-1,3-dioxo-1H-1,2,4-triazolo[1,2-a]pyridazin-5-carbonsäure vom Schmelzpunkt 151-152°C und

5-Carboxy-2,3,5,6,7,8-hexahydro-1,3-dioxo-1H-1,2,4-triazolo[1,2-a]pyridazin-2-essigsäure vom Schmelzpunkt 224-226°C.

Beispiel 23

Aus Methyl-3,5-dioxo-1,2,4-triazolidin-4-propionat und Dimethyl-hexa-2,4-diencarbonsäure unter Verwendung einer Mischung aus Chloroform und Dioxan aus Lösungsmittel erhält man in Analogie zu Beispiel 1 D) in 44%-iger Ausbeute Dimethyl-2-(2-methoxycarbonyläthyl)-2,3,5,8-tetrahydro-1,3-dioxo-1H-1,2,4-triazolo[1,2-a]pyridazin-5,8-dicarboxylat vom Schmelzpunkt 139-141°C (aus Methanol).

Die oben erhaltene Verbindung wird in 6N Salzsäure während 4 Stunden bei 100°C hydrolysiert. Durch Einengen der Lösung erhält man in 72%-iger Ausbeute 2-(2-Carboxyäthyl)-2,3,5,8-tetrahydro-1,3-dioxo-1H-1,2,4-triazolo [1,2-a]pyridazin-5,8-dicarbonsäure als hygroskopischen Festkörper.

Beispiel 24

Aus 4-(2-Chloroäthyl)-1,2,4-triazolidin-3,5-dion und 4-Phenylpenta-2,4-diencarbonsäure erhält man in Analogie zu Beispiel 1 D) in 75%-iger Ausbeute cis-2-(2-Chloräthyl)-2,3,5,8-tetrahydro-1,3-dioxo-8-phenyl-1H-1,2,4-triazolo [1,2-a]pyridazin-5-carbonsäure vom Schmelzpunkt 204-205°C. (aus Acetonitril).

Beispiel 25

Aus 4-(2-Chloräthyl)-1,2,4-triazolidin-3,5-dion und Aethyl-5-phenylpenta-2,4-diencarbonsäure erhält man in Analogie zu Beispiel 1 D) in 83%-iger Ausbeute Aethyl-cis-2-(2-chloräthyl)-2,3,5,8-tetrahydro-1,3-dioxo-8-phenyl-1H-1,2,4-triazolo[1,2-a]pyridazin-5-carboxylat vom Schmelzpunkt 130-131°C (aus Aethanol).

12,72 g (0,035 Mol) der oben erhaltenen Verbindung und 2,87 g (0,035 Mol) wasserfreies Natriumacetat werden in 100 ml Aceton während 4 Stunden unter Rückfluss zum Sieden erhitzt. Nach Entfernen des Acetons wird der Rückstand zwischen Chloroform und Wasser verteilt. Die organische Phase wird abgetrennt, über Magnesiumsulfat getrocknet und eingedampft. Man erhält in 81%-iger Ausbeute Aethyl-trans-2-(2-chloräthyl)-2,3,5,8-tetrahydro-1,3-dioxo-8-phenyl-1H-1,2,4-triazolo[1,2-a]pyridazin-5-carboxylat vom Schmelzpunkt 117-119°C (aus Toluol).

Die so erhaltene Verbindung wird in 6N Salzsäure während 4 Stunden bei 100°C hydrolysiert. Durch Einengen der erhaltenen Lösung bekommt man in 80%-iger Ausbeute trans-2-(2-chloräthyl)-2,3,5,8-tetrahydro-1,3-dioxo-8-phenyl-1H-1,2,4-triazolo[1,2-a]pyridazin-5-carbonsäure vom Schmelzpunkt 141-143°C.

Beispiel 26

Eine gekühlte Lösung von 12,08 g (0,036 Mol) cis-2-(2-Chloräthyl)-2,3,5,8-tetrahydro-1,3-dioxo-8-phenyl-1H-1,2,4-triazolo[1,2-a]pyridazin-5-carbonsäure in 180 ml Methanol wird mit einer Lösung von 2,37 g (0,036 Mol) Kaliumhydroxid in 15 ml Methanol behandelt. Nach Entfernen des Methanols wird der Rückstand in 150 ml Dimethylsulfoxid aufgenommen. Diese Lösung wird mit 4,90 g (0,043 Mol) Kaliumthioacetat versetzt und während 20 Stunden bei 80°C gerührt. Die abgekühlte Mischung wird mit 150 ml Wasser verdünnt, mit 2N Salzsäure sauer gestellt und mit Aether ausgeschüttelt. Die vereinigten Auszüge werden über Magnesiumsulfat getrocknet und zu einem gelben Oel eingedampft, das anschliessend in üblicher Weise mit Diazomethan behandelt wird. Das dabei erhaltene gelbe Oel wird an Kieselgel chromatographiert unter Eluieren mit Chloroform/Essigester (4:1), wobei man 3,8 g (27%) Methyl-trans-2-(2-acetylthioäthyl)-2,3,5,8-tetrahydro-1,3-dioxo-8-phenyl-1H-1,2,4-triazolo[1,2-a]pyridazin-5-carboxylat vom Schmelzpunkt 101-102°C (aus Essigester) und 0,1 g (1%) Methyl-cis-2-(2-acetylthioäthyl)-2,3,5,8-tetrahydro-1,3-dioxo-8-phenyl-1H-1,2,4-triazolo[1,2-a]pyridazin-5-carboxylat als farbloses Oel erhält.

Methyl-trans-2-(2-acetylthioäthyl)-2,3,5,8-tetrahydro-1,3-dioxo-8-phenyl-1H-1,2,4-triazolo[1,2-a]pyridazin-5-carboxylat wird in 2N Salzsäure während 3,5 Stunden bei 100°C hydrolysiert. Durch Einengen der erhaltenen Lösung erhält man in 43%-iger Ausbeute trans-2,3,5,8-tetrahydro-2-(2-mercaptoäthyl)-1,3-dioxo-8-phenyl-1H-1,2,4-triazolo [1,2-a]pyridazin-5-carbonsäure vom Schmelzpunkt 153-154°C.

## Beispiel 27

A) Eine Lösung von 4,73 g (0,013 Mol) Aethyl-cis-2-(2-chloräthyl)-2,3,5,8-tetrahydro-1,3-dioxo-8-phenyl-1H-1,2,4-triazolo[1,2-a]pyridazin-5-carboxylat in 300 ml Aethanol wird in der Gegenwart von 10% Palladium auf Kohle bei Raumtemperatur hydriert. Nach Abfiltrieren des Katalysators und Eindampfen erhält man 3,13 g (66%) Aethyl-2,3,5,8-hexahydro-2-(2-chloräthyl)-1,3-dioxo-8-phenyl-1H-1,2,4-triazolo[1,2-a]pyridazin-5-carboxylat vom Schmelzpunkt 87-89°C (aus Aethanol).

B) 3,6 g (0,01 Mol) des oben erhaltenen Stoffes und 1,37 g (0,012 Mol) Kaliumthioacetat werden in 160 ml Aceton während 30 Stunden unter Rückfluss zum Sieden erhitzt. Nach Entfernen des Acetons und Verteilen des Rückstandes zwischen Wasser und Essigester wird die organische Phase über Magnesiumsulfat getrocknet und eingedampft. Es resultieren 3,5 g (85%) Aethyl-2,3,5,8-hexahydro-2-(2-acetylthioäthyl)-1,3-dioxo-8-phenyl-1H-1,2,4-triazolo[1,2-a] pyridazin-5-carboxylat als gelbes Oel.

C) Die so erhaltene Verbindung wird in 2N Salzsäure während 4 Stunden bei 100°C hydrolysiert. Man erhält dabei in 77%-iger Ausbeute 2,3,5,6,7,8-Hexahydro-1-(2-mercaptoäthyl)-1,3-dioxo-8-phenyl-1H-1,2,4-triazolo[1,2-a]pyridazin-5-carbonsäure vom Schmelzpunkt 172-175°C.

Beispiel 28

Aus 4-(2-Chloräthyl)-1,2,4-triazolidin-3,5-dion und Aethyl-4-(4-chlorphenyl)penta-2,4-diencarbonsäure erhält man in Analogie zu Beispiel 1 D) in 85%-iger Ausbeute Aethyl-2-(2-chloräthyl)-2,3,5,8-terrahydro-8-(4-chlorphenyl)-1,3-dioxo-1H-1,2,4-triazolo[1,2-a]pyridazin-5-carboxylat vom Schmelzpunkt 126-127°C (aus Chloroform).

Die so erhaltene Verbindung wird in Analogie zu Beispiel 26 A) hydriert, wobei man in 80%-iger Ausbeute Aethyl-2-(2-chloräthyl)-2,3,5,6,7,8-hexahydro-8-(4-chlorphenyl)-1,3-dioxo-1H-1,2,4-triazolo[1,2-a]pyridazin-5-carboxylat vom Schmelzpunkt 111-112°C (aus Aethanol) erhält.

Der so erhaltene Stoff wird in Analogie zu Beispiel 26 B) mit Kaliumthioacetat behandelt. Man erhält in 46%-iger Ausbeute Aethyl-2-(2-acetylthioäthyl)-2,3,5,6,7,8-hexahydro-8-(4-chlorphenyl)-1,3-dioxo-1H-1,2,4-triazolo [1,2-a]pyridazin-5-carboxylat als hellgelbes Oel.

Diese Verbindung wird während 12 Stunden bei 100°C in 2N Salzsäure hydrolysiert. In 80%-iger Ausbeute erhält man 8-(4-Chlorphenyl)-2,3,5,6,7,8-hexahydro-2-(2-mercaptoäthyl)-1,3-dioxo-1H-1,2,4-triazolo[1,2-a]pyridazin-5-carbonsäure vom Schmelzpunkt 189-190°C.

Beispiel 29

Aus 4-(2-Chloräthyl)-1,2,4-triazolidin-3,5-dion und Aethyl-5-(4-methoxyphenyl)penta-2,4-diencarbonsäure erhält man in Analogie zu Beispiel 1 D) in 70%-iger Ausbeute Aethyl-cis-2-(2-chloräthyl)-2,3,5,8-tetrahydro-8-(4-nitrophenyl)-1,3-dioxo-1H-1,2,4-triazolo[1,2-a]pyridazin-5-carboxylat vom Schmelzpunkt 110-112°C (aus Aethanol).

Diese Verbindung wird in 6N Salzsäure während 10 Stunden bei 100°C hydrolysiert, wobei man in 30%-iger Ausbeute cis-2-(2-chloräthyl)-2,3,5,8-tetrahydro-8-(4-methoxyphenyl)-1,3-dioxo-1H-1,2,4-triazolo[1,2-a]pyridazin-5-carbonsäure vom Schmelzpunkt 158-161°C (aus Essigester/Hexan) erhält.

## Beispiel 30

Aus 4-(2-Chloräthyl)-1,2,4-triazolidin-3,5-dion und Aethyl-5-(4-nitrophenyl)penta-2,4-diencarbonsäure erhält man in Analogie zu Beispiel 1 D) in 75%-iger Ausbeute Aethyl-cis-2-(2-chloräthyl)-2,3,5,8-tetrahydro-8-(4-nitrophenyl)-1,3-dioxo-1H-1,2,4-triazolo[1,2-a]pyridazin-5-carboxylat vom Schmelzpunkt 142-143°C (aus Toluol).

Durch Hydrolyse der so erhaltenen Verbindung in 6N Salzsäure während 12 Stunden bei 100°C erhält man in 34%-iger Ausbeute cis-2-(2-chloräthyl)-2,3,5,8-tetrahydro-8-(4-nitrophenyl)-1,3-dioxo-1H-1,2,4-triazolo[1,2-a]pyridazin-5-carbonsäure vom Schmelzpunkt 192-194°C (aus Essigester).

## Beispiel 31

Aus 1,2,4-Triazolidin-3,5-dion und Aethyl-5-phenylpenta-2,4-diencarbonsäure erhält man in Analogie zu Beispiel 1 D) in 81%-iger Ausbeute Aethyl-2,3,5,8-tetrahydro-1,3-dioxo-8-phenyl-1H-1,2,4-triazolo[1,2-a]pyridazin-5-carboxylat vom Schmelzpunkt 196-197°C (aus Aethanol).

Nach Behandeln dieses Stoffes mit Formaldehyd in Analogie zu Beispiel 18 erhält man in 60%-iger Ausbeute Aethyl-2-hydroxymethyl-2,3,5,8-tetrahydro-1,3-dioxo-8-phenyl-1H-1,2,4-triazolo[1,2-a]pyridazin-5-carboxylat vom Schmelzpunkt 129-133°C (aus Essigester/Hexan).

Diese Verbindung wird in Analogie zu Beispiel 18 mit Phosphorpentachlorid umgesetzt. Es resultiert dabei in 73%-iger Ausbeute Aethyl-2-chlormethyl-2,3,5,8-tetrahydro-1,3-dioxo-8-phenyl-1H-1,2,4-triazolo[1,2-a]pyridazin-5-carboxylat vom Schmelzpunkt 145-146°C (aus Essigester).

Nach Hydrieren der so erhaltenen Verbindung in Analogie zu Beispiel 26 A) erhält man in 64%-iger Ausbeute Aethyl-2-chlormethyl-2,3,5,6,7,8-hexahydro-1,3-dioxo-8-phenyl-1H-1,2,4-triazolo[1,2-a]pyridazin-5-carboxylat vom Schmelzpunkt 104-106°C (aus Essigester/Hexan).

Diese Verbindung wird in Analogie zu Beispiel 10 mit Kaliumthioacetat umgesetzt. Man erhält in 80%-iger Ausbeute Aethyl-2-acetylthiomethyl-2,3,5,6,7,8-hexahydro-1,3-dioxo-8-phenyl-1H-1,2,4-triazolo[1,2-a]pyridazin-5-carboxylat als schwach gelbes Oel.

Nach Hydrolyse dieses Stoffes in 2N Salzsäure bei 100°C während 2,5 Stunden und Eindampfen der erhaltenen Lösung erhält man in 81%-iger Ausbeute 2,3,5,6,7,8-Hexahydro-2-mercaptomethyl-1,3-dioxo-8-phenyl-1H-1,2,4-triazolo[1,2-a]pyridazin-5-carbonsäure vom Schmelzpunkt 85-86°C (Zersetzung).

## Beispiel 32

Eine Mischung aus 440 mg Methyl-2-acetylthiomethyl-2,3,5,8-tetrahydro-1,3-dioxo-1H-1,2,4-triazolo[1,2-a]pyridazin-5-carboxylat, 3 ml Methanol und 1N Natronlauge wird unter einer Stickstoffatmosphäre während 2 Stunden bei 20°C gerührt. Die erhaltene Lösung wird angesäuert, mit Natriumchlorid gesättigt und mit Chloroform ausgeschüttelt. Die Chloroform-Auszüge werden über Magnesiumsulfat getrocknet und eingedampft. Nach Umkristallisieren aus Essigester/Hexan resultieren 220 mg (62%) 2,3,5,8-Tetrahydro-2-mercaptomethyl-1,3-dioxo-1H-1,2,4-triazolo[1,2-a]pyridazin-5-carbonsäure vom Schmelzpunkt 185-187°C.

Beispiel 33

Aus Methyl-2,3,5,8-tetrahydro-2-(3-chlorpropyl)-1,3-dioxo-1H-1,2,4-triazolo[1,2-a]pyridazin-5-carboxylat erhält man in Analogie zu Beispiel 10 in 40%-iger Ausbeute 2,3,5,8-Tetrahydro-2-(3-acetylthiopropyl)-1,3-dioxo-1H-1,2,4-triazolo[1,2-a]pyridazin-5-carboxylat als Oel.

In Analogie zu Beispiel 32 erhält man aus Methyl-2,3,5,8-tetrahydro-2-(3-acetylthiopropyl)-1,3-dioxo-1H-1,2,4-triazolo[1,2-a]pyridazin-5-carboxylat in 35%-iger Ausbeute 2,3,5,8-Tetrahydro-2-(3-mercaptopropyl)-1,3-dioxo-1H-1,2,4-triazolo[1,2-a]pyridazin-5-carbonsäure vom Schmelzpunkt 119-121°C (aus Essigester/Hexan).

Beispiel 34

Methyl-2-chlormethyl-2,3,5,8-tetrahydro-1,3-dioxo-1H-1,2,4-triazolo[1,2-a]pyridazin-5-carboxylat wird in Analogie zu Beispiel 16 in Methyl-2-chlormethyl-2,3,5,6,7,8-hexahydro-1,3-dioxo-1H-1,2,4-triazolo[1,2-a]pyridazin-5-carboxylat vom Schmelzpunkt 125-127°C überführt.

Aus Methyl-2-chlormethyl-2,3,5,6,7,8-hexahydro-1,3-dioxo-1H-1,2,4-triazolo[1,2-a]pyridazin-5-carboxylat erhält man in Analogie zu Beispiel 10 in 37%-iger Ausbeute Methyl-2-acetylthiomethyl-2,3,5,6,7,8-hexahydro-1,3-dioxo-1H-1,2,4-triazolo[1,2-a]pyridazin-5-carboxylat vom Schmelzpunkt 100-102°C (aus Essigester/Hexan).

Aus Methyl-2-acetylthiomethyl-2,3,5,6,7,8-hexahydro-1,3-dioxo-1H-1,2,4-triazolo[1,2-a]pyridazin-5-carboxylat erhält man in Analogie zu Beispiel 32 in 29%-iger Ausbeute 2,3,5,6,7,8-Hexahydro-2-mercaptomethyl-1,3-dioxo-1H-1,2,4-triazolo[1,2-a]pyridazin-5-carbonsäure vom

0024309

Schmelzpunkt 157,5-158,5°C (aus Essigester/Hexan).

Die folgenden Beispiele erläutern die Herstellung pharmazeutischer Präparate, enthaltend Triazolopyridazin-Derivate gemäss vorliegender Erfindung:

## Beispiel A

Tabletten folgender Zusammensetzung werden in an sich üblicher Weise hergestellt:

| Bestandteile | Pro Tablette |
|---|---|
| Triazolopyridazin-Derivate | 10,0 mg |
| Lactose | 125,0 mg |
| Mais-Stärke | 75,0 mg |
| Talk | 4,0 mg |
| Magnesiumstearat | 1,0 mg |
| Gesamtgewicht | 215,0 mg |

## Beispiel B

Kapseln folgender Zusammensetzung werden in an sich üblicher Weise hergestellt:

| Bestandteile | Pro Kapsel |
|---|---|
| Triazolopyridazin-Derivat | 25,0 mg |
| Lactose | 150,0 mg |
| Mais-Stärke | 20,0 mg |
| Talk | 5,0 mg |
| Gesamtgewicht | 200,0 mg |

## Patentansprüche

1. Triazolopyridazin-Derivate der allgemeinen Formel

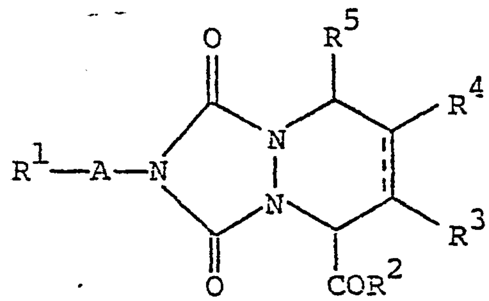

I

worin A eine gegebenenfalls durch niederes Alkyl substituierte Methylen-, Aethylen- oder Propylen-Gruppe bedeutet, $R^1$ Halogen, Carboxyl, niederes Alkoxycarbonyl, Aminocarbonyl, Hydroxyaminocarbonyl, Hydrazinocarbonyl, Mercapto, niederes Alkanoylthio oder Aryl-(niederes Alkylthio) und $R^2$ Hydroxy, niederes Alkoxy oder Amino bedeuten, und entweder $R^3$ und $R^4$ Wasserstoff oder Halogen bedeuten, oder $R^3$ Wasserstoff und $R^4$ Hydroxy bedeuten, $R^5$ Wasserstoff, niederes Alkyl, Aryl, Carboxy, niederes Alkoxycarbonyl oder Aminocarbonyl bedeutet und wenn $R^3$ und $R^4$ beide Wasserstoff bedeuten, die gestrichelte Linie eine fakultative Kohlenstoff-Kohlenstoff-Bindung bedeutet,

und Salze von Carbonsäuren der allgemeinen Formel I mit pharmazeutisch annehmbaren Basen.

2. Triazolopyridazin-Derivate gemäss Anspruch 1, worin A eine Aethylen-Gruppe bedeutet.

3. Triazolopyridazin-Derivate gemäss Anspruch 1 oder 2, worin $R^1$ Carboxyl, Hydroxyaminocarbonyl oder Mercapto bedeutet.

4. Triazolopyridazin-Derivate gemäss einem der Ansprüche 1 bis 3, worin $R^2$ Hydroxy bedeutet.

5. Triazolopyridazin-Derivate gemäss einem der Ansprüche 1 bis 4, worin in 6,7-Stellung eine Doppel-Bindung vorhanden ist.

6. Triazolopyridazin-Derivate gemäss einem der Ansprüche 1 bis 5, worin $R^4$ Wasserstoff oder Aryl bedeutet.

7. 2,3,5,8-Tetrahydro-2-(2-mercaptoäthyl)-1,3-dioxo-1H-1,2,4-triazolo[1,2-a]pyridazin-5-carbonsäure.

8. 5-Carboxy-2,3,5,8-tetrahydro-1,3-dioxo-1H-1,2,4-triazolo[1,2-a]pyridazin-2-propionsäure.

9. 2-[2-(N-Hydroxycarbamoyl)äthyl]-2,3,5,8-tetrahydro-1,3-dioxo-1H-1,2,4-triazolo[1,2-a]pyridazin-5-carbonsäure.

10. 5-Carboxy-2,3,5,8-tetrahydro-8-phenyl-1,3-dioxo-1H-1,2,4-triazolo[1,2-a]pyridazin-2-propionsäure.

11. 5-Carboxy-2,3,5,8-tetrahydro-1,3-dioxo-1H-1,2,4-triazolo[1,2-a]pyridazin-2-essigsäure, 5-Carboxy-2,3,5,8-tetrahydro-1,3-dioxo-1H-1,2,4-triazolo[1,2-a]pyridazin-2-buttersäure, 2-(2-Chloräthyl)-2,3,5,8-tetrahydro-1,3-dioxo-1H-1,2,4-triazolo[1,2-a]pyridazin-5-carbonsäure, 2-(3-Chlorpropyl)-2,3,5,8-tetrahydro-1,3-dioxo-1H-1,2,4-triazolo[1,2-a]pyridazin-5-carbonsäure, Methyl-5-carboxy-2,3,5,8-tetrahydro-1,3-dioxo-1H-1,2,4-triazolo[1,2-a]pyridazin-2-propionat, 5-Carboxy-2,3,5,8-tetrahydro-8-methyl-1,3-dioxo-1H-1,2,4-triazolo[1,2-a]pyridazin-2-propionsäure, 2-(2-Benzylthioäthyl)-2,3,5,8-tetrahydro-1,3-dioxo-1H-1,2,4-triazolo[1,2-a]pyridazin-5-carbonsäure, Methyl-2-acetylthiomethyl-2,3,5,8-tetrahydro-1,3-dioxo-1H-1,2,4-triazolo[1,2-a]pyridazin-5-carboxylat, t-Butyl-2-(2-acetylthioäthyl)-2,3,5,8-tetrahydro-1,3-dioxo-1H-1,2,4-triazolo[1,2-a]pyridazin-5-carboxylat, t-Butyl-2-[2-(N-hydroxy-

carbamoyl)äthyl]-2,3,5,8-tetrahydro-1,3-dioxo-1H-1,2,4-triazolo[1,2-a]pyridazin-5-carboxylat, 2-(2-Carbamoyläthyl)-2,3,5,8-tetrahydro-1,3-dioxo-1H-1,2,4-triazolo[1,2-a]pyridazin-5-carbonsäure, 5-Carbamoyl-2,3,5,8-tetrahydro-1,3-dioxo-1H-1,2,4-triazolo[1,2-a]pyridazin-2-propionamid, 2-[(2-Hydrazinocarbonyl)äthyl]-2,3,5,8-tetrahydro-1,3-dioxo-1H-1,2,4-triazolo[1,2-a]pyridazin-5-carbonsäure, 5-Carboxy-2,3,5,6,7,8-hexahydro-1,3-dioxo-1H-1,2,4-triazolo[1,2-a]pyridazin-2-essigsäure, 5-Carboxy-2,3,5,6,7,8-hexahydro-1,3-dioxo-1H-1,2,4-triazolo[1,2-a]pyridazin-2-propionsäure, 5-Carboxy-2,3,5,6,7,8-hexahydro-1,3-dioxo-1H-1,2,4-triazolo[1,2-a]pyridazin-2-buttersäure, Methyl-2,3,5,6,7,8-hexahydro-1,3-dioxo-1H-1,2,4-triazolo[1,2-a]pyridazin-2-propionat, 2-(2-Chloräthyl)-2,3,5,6,7,8-hexahydro-1,3-dioxo-1H-1,2,4-triazolo[1,2-a]pyridazin-5-carbonsäure, Benzyl-5-methoxycarbonyl-2,3,5,8-tetrahydro-1,3-dioxo-1H-1,2,4-triazolo[1,2-a]pyridazin-2-acetat, Methyl-2-(2-chloräthyl)-2,3,5,6,7,8-hexahydro-1,3-dioxo-1H-1,2,4-triazolo[1,2-a]pyridazin-5-carboxylat, Methyl-2-chlormethyl-2,3,5,8-tetrahydro-1,3-dioxo-1H-1,2,4-triazolo[1,2-a]pyridazin-5-carboxylat, 6,7-Dibrom-5-carboxy-hexahydro-1,3-dioxo-1H-1,2,4-triazolo[1,2-a]pyridazin-2-propionsäure, 5-Carboxy-hexahydro-7-hydroxy-1,3-dioxo-1H-1,2,4-triazolo[1,2-a]pyridazin-2-propionsäure, t-Butyl-2-(2-chloräthyl)-2,3,5,8-tetrahydro-1,3-dioxo-1H-1,2,4-triazolo[1,2-a]pyridazin-5-carboxylat, Methyl-2,3,5,8-tetrahydro-5-t-butoxycarbonyl-1,3-dioxo-1H-1,2,4-triazolo[1,2-a]pyridazin-2-propionat und Methyl-2,3,5,8-tetrahydro-5-methoxycarbonyl-1,3-dioxo-1H-1,2,4-triazolo[1,2-a]pyridazin-2-propionat.

12. Dimethyl-2-(2-methoxycarbonyläthyl)-2,3,5,8-tetrahydro-1,3-dioxo-1H-1,2,4-triazolo[1,2-a]pyridazin-5,8-dicarboxylat, 2-(2-Carboxyäthyl)-2,3,5,8-tetrahydro-1,3-dioxo-1H-1,2,4-triazolo[1,2-a]pyridazin-5,8-dicarbonsäure, cis-2-(2-Chloräthyl)-2,3,5,8-tetrahydro-1,3-dioxo-8-phenyl-1H-1,2,4-triazolo[1,2-a]pyridazin-5-carbonsäure, Aethyl-cis-2-(2-chloräthyl)-2,3,5,8-tetrahydro-1,3-dioxo-8-phenyl-1H-1,2,4-triazolo[1,2-a]pyridazin-5-carboxylat, Aethyl-trans-2-(2-chloräthyl)-2,3,5,8-tetrahydro-1,3-dioxo-8-phenyl-1H-1,2,4-triazolo[1,2-a]pyridazin-5-carboxylat, trans-2-(2-Chloräthyl)-2,3,5,8-tetrahydro-1,3-dioxo-8-phenyl-1H-1,2,4-triazolo[1,2-a]pyridazin-5-carbonsäure, Methyl-trans-2-(2-acetylthioäthyl)-2,3,5,8-tetrahydro-1,3-dioxo-8-phenyl-1H-1,2,4-triazolo[1,2-a]pyridazin-5-carboxylat, Methyl-cis-2-(2-acetylthioäthyl)-2,3,5,8-tetrahydro-1,3-dioxo-8-phenyl-1H-1,2,4-triazolo [1,2-a]pyridazin-5-carboxylat, trans-2,3,5,8-Tetrahydro-2-(2-mercaptoäthyl)-1,3-dioxo-8-phenyl-1H-1,2,4-triazolo [1,2-a]pyridazin-5-carbonsäure, Aethyl-2,3,5,6,7,8-hexahydro-2-(2-chloräthyl)-1,3-dioxo-8-phenyl-1H-1,2,4-triazolo[1,2-a]pyridazin-5-carboxylat, Aethyl-2,3,5,6,7,8-hexahydro-2-(2-acetylthioäthyl)-1,3-dioxo-8-phenyl-1H-1,2,4-triazolo[1,2-a]pyridazin-5-carboxylat, 2,3,5,6,7,8-Hexahydro-2-(2-mercaptoäthyl)-1,3-dioxo-8-phenyl-1H-1,2,4-triazolo[1,2-a]pyridazin-5-carbonsäure, Aethyl-2-(2-chloräthyl)-2,3,5,8-tetrahydro-8-(4-chlorphenyl)-1,3-dioxo-1H-1,2,4-triazolo[1,2-a]pyridazin-5-carboxylat, Aethyl-2-(2-chloräthyl)-2,3,5,6,7,8-hexahydro-8-(4-chlorphenyl)-1,3-dioxo-1H-1,2,4-triazolo[1,2-a]pyridazin-5-carboxylat, Aethyl-2-(2-acetylthioäthyl)-2,3,5,6,7,8-hexahydro-8-(4-chlorphenyl)-1,3-dioxo-1H-1,2,4-triazolo[1,2-a]-pyridazin-5-carboxylat, 8-(4-Chlorphenyl)-2,3,5,6,7,8-hexahydro-2-(2-mercaptoäthyl)-1,3-dioxo-1H-1,2,4-triazolo [1,2-a]-pyridazin-5-carbonsäure, Aethyl-cis-2-(2-chloräthyl)-2,3,5,8-tetrahydro-8-(4-methoxyphenyl)-1,3-dioxo-1H-1,2,4-triazolo[1,2-a]-pyridazin-5-carboxylat, cis-2-(2-Chloräthyl)-2,3,5,8-tetrahydro-8-(4-methoxyphenyl)-1,3-dioxo-1H-1,2,4-triazolo[1,2-a]-pyridazin-5-carbonsäure, Aethyl-cis-2-(2-chloräthyl)-2,3,5,8-tetrahydro-8-(4-nitrophenyl)-1,3-dioxo-1H-1,2,4-triazolo[1,2-a]-pyridazin-5-carboxylat, cis-2-(2-Chloräthyl)-2,3,5,8-tetrahydro-8-(4-nitrophenyl)-1,3-dioxo-1H-1,2,4-triazolo[1,2-a]pyridazin-5-carbonsäure, Aethyl-2-chlormethyl-1,3,5,8-tetrahydro-1,3-dioxo-8-phenyl-1H-1,2,4-triazolo[1,2-a]pyridazin-5-carboxalat, Aethyl-2-chlormethyl-2,3,5,6,7,8-hexahydro-1,3-dioxo-8-phenyl-1H-1,2,4-triazolo[1,2-a]pyridazin-5-carboxylat, Aethyl-2-acetylthiomethyl)-2,3,5,6,7,8-hexahydro-1,3-dioxo-8-phenyl-1H-1,2,4-triazolo[1,2-a]pyridazin-5-carboxylat, 2,3,5,6,7,8-Hexahydro-2-mercaptomethyl-1,3-dioxo-8-phenyl-1H-1,2,4-triazolo[1,2-a]pyridazin-5-carbonsäure, 2,3,5,8-Tetrahydro-2-mercaptomethyl-1,3-dioxo-1H-1,2,4-triazolo[1,2-a]pyridazin-5-carbonsäure, Methyl-2,3,5,8-tetrahydro-2-(3-acetylthiopropyl)-1,3-dioxo-1H-1,2,4-triazolo[1,2-a]pyridazin-5-carboxylat, 2,3,5,8-Tetrahydro-2-(3-mercaptopropyl)-1,3-dioxo-1H-1,2,4-triazolo [1,2-a]pyridazin-5-carbonsäure, Methyl-2-chlormethyl-2,3,5,6,7,8-hexahydro-1,3-dioxo-1H-1,2,4-triazolo[1,2-a] pyridazin-5-carboxylat, Methyl-2-acetylthiomethyl)-2,3,5,6,7,8-hexahydro-1,3-dioxo-1H-1,2,4-triazolo[1,2-a] pyridazin-5-carbonsäure 2,3,5,6,7,8-Hexahydro-2-mercaptomethyl-1,3-dioxo-1H-1,2,4-triazolo[1,2-a]pyridazin-5-carbonsäure.

13. Verbindungen der allgemeinen Formel

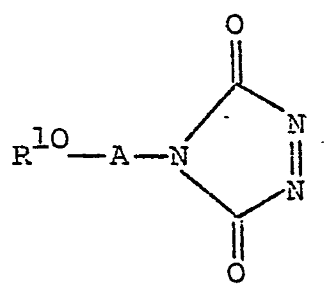

II

worin A eine gegebenenfalls durch niederes Alkyl substituierte Methylen-, Aethylen- oder Propylen-Gruppe und $R^{10}$ Halogen, Carboxyl oder niederes Alkoxycarbonyl bedeuten.

14. Verbindungen der allgemeinen Formel

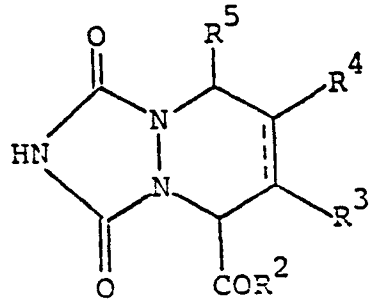

VI

worin $R^2$ Hydroxy, niederes Alkoxy oder Amino bedeutet, und entweder $R^3$ und $R^4$ je Wasserstoff oder Halogen bedeuten, oder $R^3$ Wasserstoff und $R^4$ Hydroxy bedeuten, $R^5$ Wasserstoff, niederes Alkyl, Aryl, Carboxy, niederes Alkoxycarbonyl oder Aminocarbonyl bedeutet, und, wenn $R^3$ und $R^4$ beide Wasserstoff bedeuten, die gestrichelte Linie eine fakultative Kohlenstoff-Kohlenstoff-Bindung bedeutet, mit der Massgabe, dass $R^5$ nicht Wasserstoff bedeutet, wenn in 6,7-Stellung eine Doppelbindung vorhanden ist und $R^2$ Hydroxy bedeutet.

15. Verbindungen der allgemeinen Formel

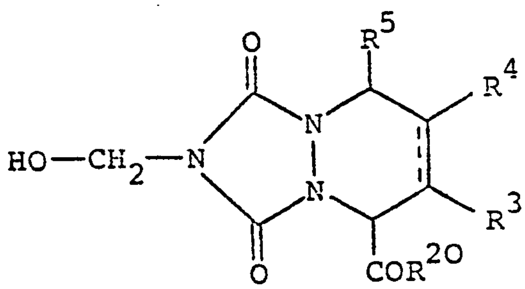

VIII

worin $R^{20}$ Hydroxy, niederes Alkoxy oder Amino bedeutet, und entweder $R^3$ und $R^4$ je Wasserstoff oder Halogen bedeuten, oder $R^3$ Wasserstoff und $R^4$ Hydroxy bedeuten, $R^5$ Wasserstoff, niederes Alkyl, Aryl, Carboxy, niederes Alkoxycarbonyl oder Aminocarbonyl bedeutet und, wenn $R^3$ und $R^4$ beide Wasserstoff bedeuten, die gestrichelte Linie eine fakultative Kohlenstoff-Kohlenstoff-Bindung bedeutet.

16. Verbindungen der allgemeinen Formel

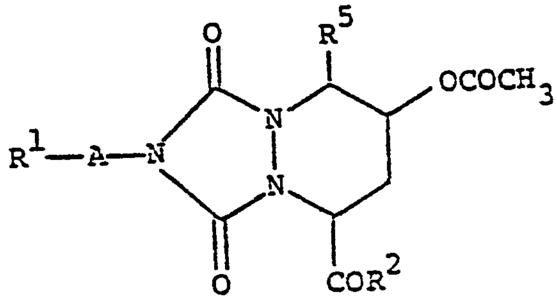

XI

worin A eine gegebenenfalls durch niederes Alkyl substituierte Methylen-, Aethylen- oder Propylen-Gruppe, $R^1$ Halogen, Carboxyl, niederes Alkoxycarbonyl, Aminocarbonyl, Hydroxyaminocarbonyl, Hydrazinocarbonyl, Mercapto, niederes Alkanoylthio oder Aryl-(niederes Alkylthio) und $R^2$ Hydroxy, niederes Alkoxy oder Amino bedeuten, $R^5$ Wasserstoff, niederes Alkyl, Aryl, Carboxy, niederes Alkoxycarbonyl oder Aminocarbonyl bedeutet.

17. Verbindungen der allgemeinen Formel

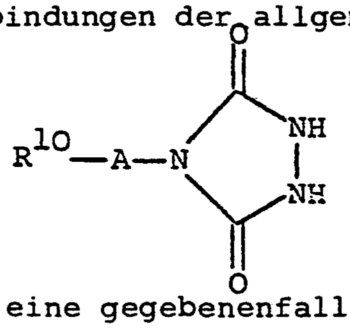

XIV

worin A eine gegebenenfalls durch niederes Alkyl substituierte Methylen-, Aethylen- oder Propylen-Gruppe und $R^{10}$ Halogen, Carboxyl oder niederes Alkoxycarbonyl bedeuten.

18. Triazolopyridazin-Derivate gemäss einem der Ansprüche 1 bis 12 oder pharmazeutisch annehmbare Salze von Carbonsäuren gemäss einem der Ansprüche 1 bis 12 als pharmazeutische Wirkstoffe.

19. Triazolopyridazin-Derivate gemäss einem der Ansprüche 1 bis 12 oder pharmazeutisch annehmbare Salze von Carbonsäuren gemäss einem der Ansprüche 1 bis 12 als Antihypertensiva.

20. Verfahren zur Herstellung von Triazolopyridazin-Derivaten der allgemeinen Formel I gemäss Anspruch 1, dadurch gekennzeichnet, dass man

(a) zur Herstellung von Verbindungen der allgemeinen Formel I, worin $R^1$ Halogen, Carboxyl oder niederes Alkoxycarbonyl bedeutet, und in 6,7-Stellung eine Doppelbindung vorhanden ist und A, $R^2$ und $R^5$ die in Anspruch 1 angegebene Bedeutung besitzen, eine Verbindung der allgemeinen Formel

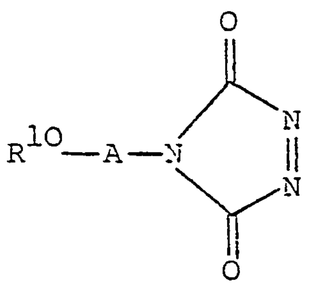

II

worin $R^{10}$ Halogen, Carboxyl oder niederes Alkoxycarbonyl bedeutet,

mit einer Verbindung der allgemeinen Formel

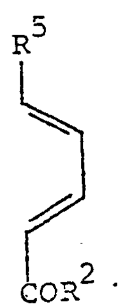

III

worin $R^2$ und $R^5$ die in Anspruch 1 angegebene Bedeutung besitzen,

umsetzt, oder

(b) zur Herstellung von Verbindungen der allgemeinen Formel I, worin $R^1$ niederes Alkanoylthio oder Aryl-(niederes Alkylthio) bedeutet und A, $R^2$, $R^3$, $R^4$, $R^5$ und die

gestrichelte Linie die in Anspruch 1 angegebene Bedeutung besitzen, eine Verbindung der allgemeinen Formel

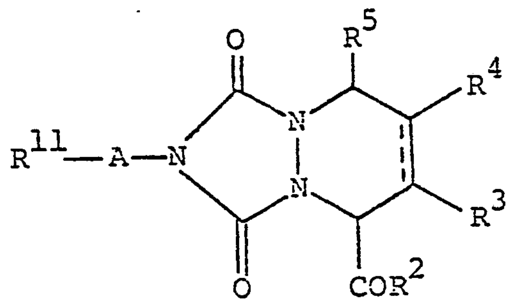

Ia

worin A, $R^2$, $R^3$, $R^4$, $R^5$ und die gestrichelte Linie die in Anspruch 1 angegebene Bedeutung besitzen, und $R^{11}$ Halogen bedeutet,

mit einer Verbindung der allgemeinen Formel

$$R^6\text{-SH} \qquad \text{IV}$$

worin $R^6$ niederes Alkanoyl oder Aryl-(niederes Alkyl) bedeutet,

umsetzt, oder

(c) zur Herstellung von Verbindungen der allgemeinen Formel I, worin $R^1$ Mercapto bedeutet, und A, $R^2$, $R^3$, $R^4$, $R^5$ und die gestrichelte Linie die in Anspruch 1 angegebene Bedeutung besitzen, in einer Verbindung der allgemeinen Formel

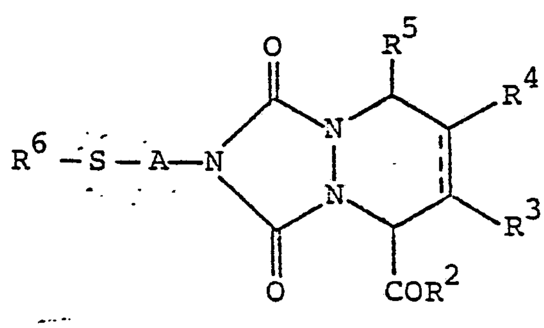

Ib

worin A, $R^2$, $R^5$ und die gestrichelte Linie die in Anspruch 1 angegebene Bedeutung besitzen, und $R^6$ obige Bedeutung besitzt,

die mit $R^6$ bezeichnete Gruppe abspaltet, oder

(d) zur Herstellung von Verbindungen der allgemeinen Formel I, worin $R^1$ Aminocarbonyl, Hydroxyaminocarbonyl oder Hydrazinocarbonyl bedeutet, und A, $R^2$, $R^3$, $R^4$, $R^5$ und die gestrichelte Linie die in Anspruch 1 angegebene Bedeutung besitzen, eine Verbindung der allgemeinen Formel

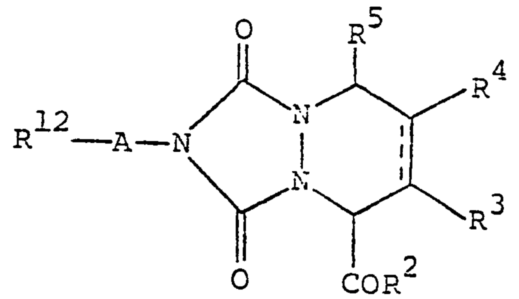

Ic

worin A, $R^2$, $R^3$, $R^4$, $R^5$ und die gestrichelte Linie die in Anspruch 1 angegebene Bedeutung besitzen, und $R^{12}$ niederes Alkoxycarbonyl bedeutet,

mit einer Verbindung der allgemeinen Formel

$$Y\text{-}NH_2 \qquad V$$

worin Y Wasserstoff, Hydroxy oder Amino bedeutet,

umsetzt, oder

(e) zur Herstellung von Verbindungen der allgemeinen Formel I, worin $R^1$ Halogen, Carboxyl, niederes Alkoxycarbonyl, Aminocarbonyl, Hydroxyaminocarbonyl oder Hydrazinocarbonyl, $R^3$ und $R^4$ beide Wasserstoff bedeuten, in 6,7-Stellung eine Einfachbindung vorhanden ist, und A, $R^2$ und $R^5$ die in Anspruch 1 angegebene Bedeutung besitzen, eine entsprechende Verbindung der allgemeinen Formel I, worin in 6,7-Stellung eine Doppelbindung vorhanden ist, katalytisch hydriert, oder

(f) eine Verbindung der allgemeinen Formel

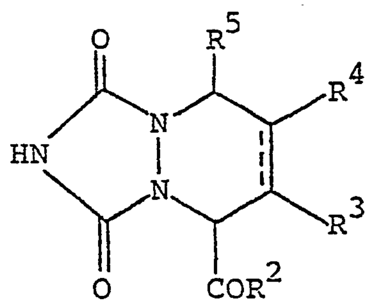

VI

worin $R^2$, $R^3$, $R^4$, $R^5$ und die gestrichelte Linie die in Anspruch 1 angegebene Bedeutung besitzen,
mit einer Verbindung der allgemeinen Formel

$R^1$-A-X VII

worin $R^1$ und A die in Anspruch 1 angegebene Bedeutung besitzen und X eine Abgangsgruppe bedeutet,
umsetzt, oder

(g) zur Herstellung von Verbindungen der allgemeinen Formel I, worin A eine Methylen-Gruppe, $R^1$ Halogen und $R^2$ niederes Alkoxy oder Amino bedeuten und $R^3$, $R^4$, $R^5$ und die gestrichelte Linie die in Anspruch 1 angegebene Bedeutung besitzen, eine Verbindung der allgemeinen Formel

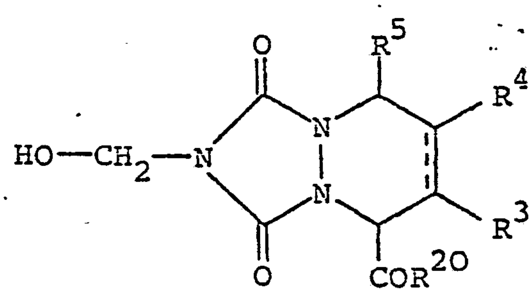

VIII

worin $R^3$, $R^4$, $R^5$ und die gestrichelte Linie die in Anspruch 1 angegebene Bedeutung besitzen, und $R^{20}$ niederes Alkoxy oder Amino bedeutet,
geeignet halogeniert, oder

(h) zur Herstellung von Verbindungen der allgemeinen

Formel I, worin $R^3$ und $R^4$ beide Halogen bedeuten, in 6,7-Stellung eine Einfachbindung vorhanden ist, und A, $R^1$, $R^2$ und $R^5$ die in Anspruch 1 angegebene Bedeutung besitzen, eine Verbindung der allgemeinen Formel I, worin in 6,7-Stellung eine Doppelbindung vorhanden ist, mit einem Halogenwasserstoff in Gegenwart von Wasserstoffperoxid behandelt, oder

(i) zur Herstellung von Verbindungen der allgemeinen Formel I, worin $R^3$ Wasserstoff und $R^4$ Hydroxy bedeuten, in 6,7-Stellung eine Einfachbindung vorhanden ist, und A, $R^1$, $R^2$ und $R^5$ die in Anspruch 1 angegebene Bedeutung besitzen, eine Verbindung der allgemeinen Formel

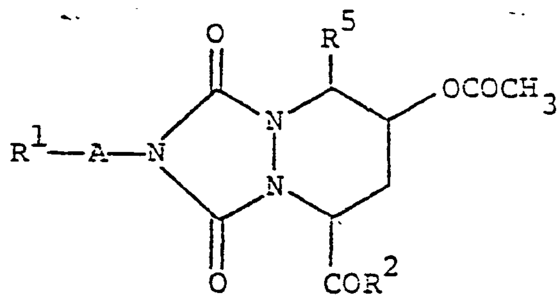

XI

worin A, $R^1$, $R^2$ und $R^5$ die in Anspruch 1 angegebene Bedeutung besitzen,

hydrolysiert, oder

(j) zur Herstellung von Verbindungen der allgemeinen Formel I, worin $R^1$ niederes Alkoxycarbonyl und/oder $R^2$ niederes Alkoxy bedeutet, eine entsprechende Verbindung der allgemeinen Formel I, worin $R^1$ Carboxyl und/oder $R^2$ Hydroxy bedeuten, verestert, oder

(k) zur Herstellung von Verbindungen der allgemeinen Formel I, worin $R^1$ Carboxyl und/oder $R^2$ Hydroxy bedeutet, eine entsprechende Verbindung der allgemeinen Formel I, worin $R^1$ niederes Alkoxycarbonyl und/oder $R^2$ niederes Alkoxy bedeutet, mit einer Säure oder einer Base behandelt, oder

(l) zur Herstellung eines Salzes einer Carbonsäure der allgemeinen Fomel I, eine Carbonsäure der allgemeinen Formel I in ein pharmazeutisch annehmbares Salz mit einer Base überführt.

21. Arzneimittel, enthaltend ein Triazolopyridazin-Derivat gemäss einem der Ansprüche 1 bis 12 oder ein pharmazeutisch annehmbares Salz einer Carbonsäure gemäss einem der Ansprüche 1 bis 12.

22. Antihypertensiva, enthaltend ein Triazolopyridazin-Derivat gemäss einem der Ansprüche 1 bis 12 oder ein pharmazeutisch annehmbares Salz einer Carbonsäure gemäss einem der Ansprüche 1 bis 12.

23. Die Verwendung von Triazolopyridazin-Derivaten gemäss einem der Ansprüche 1 bis 12 oder von pharmazeutisch annehmbaren Salzen von Carbonsäuren gemäss einem der Ansprüche 1 bis 12 bei der Bekämpfung bzw. Verhütung von Krankheiten.

24. Die Verwendung von Triazolopyridazin-Derivaten gemäss einem der Ansprüche 1 bis 12 oder von pharmazeutisch annehmbaren Salzen von Carbonsäuren gemäss einem der Ansprüche 1 bis 12 bei der Bekämpfung bzw. Verhütung von Hypertensionen.

***

Patentansprüche

"OESTERREICH"

1. Verfahren zur Herstellung von Triazolopyridazin-Derivaten der allgemeinen Formel

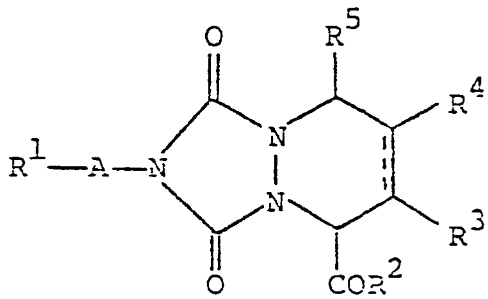

I

worin A eine gegebenenfalls durch niederes Alkyl substituierte Methylen-, Aethylen- oder Propylen-Gruppe bedeutet, $R^1$ Halogen, Carboxyl, niederes Alkoxycarbonyl, Aminocarbonyl, Hydroxyaminocarbonyl, Hydrazinocarbonyl, Mercapto, niederes Alkanoylthio oder Aryl-(niederes Alkylthio) und $R^2$ Hydroxy, niederes Alkoxy oder Amino bedeuten, und entweder $R^3$ und $R^4$ Wasserstoff oder Halogen bedeuten, oder $R^3$ Wasserstoff und $R^4$ Hydroxy bedeuten, $R^5$ Wasserstoff, niederes Alkyl, Aryl, Carboxy, niederes Alkoxycarbonyl oder Aminocarbonyl bedeutet und, wenn $R^3$ und $R^4$ beide Wasserstoff bedeuten, die gestrichelte Linie eine fakultative Kohlenstoff-Kohlenstoff-Bindung bedeutet,

und Salze von Carbonsäuren der allgemeinen Formel I mit pharmazeutisch annehmbaren Basen, dadurch gekennzeichnet, dass man

(a) zur Herstellung von Verbindungen der allgemeinen Formel I, worin $R^1$ Halogen, Carboxyl oder niederes Alkoxycarbonyl bedeutet, und in 6,7-Stellung eine Doppelbindung vorhanden ist und A, $R^2$ und $R^5$ obige Bedeutung besitzen, eine Verbindung der allgemeinen Formel

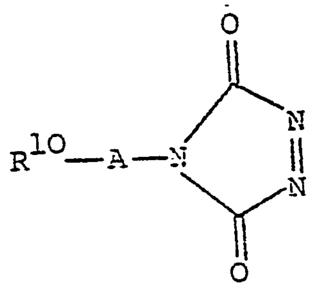

II

worin $R^{10}$ Halogen, Carboxyl oder niederes Alkoxycarbonyl bedeutet,

mit einer Verbindung der allgemeinen Formel

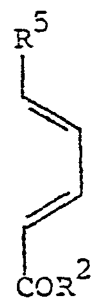

III

worin $R^2$ und $R^5$ obige Bedeutung besitzen,

umsetzt, oder

(b) zur Herstellung von Verbindungen der allgemeinen Formel I, worin $R^1$ niederes Alkanoylthio oder Aryl-(niederes Alkylthio) bedeutet und A, $R^2$, $R^3$, $R^4$, $R^5$ und die gestrichelte Linie obige Bedeutung besitzen, eine Verbindung der allgemeinen Formel

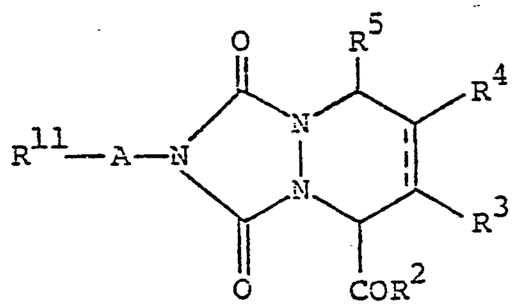

Ia

worin A, $R^2$, $R^3$, $R^4$, $R^5$ und die gestrichelte Linie obige Bedeutung besitzen, und $R^{11}$ Halogen bedeutet,

mit einer Verbindung der allgemeinen Formel

$R^6$-SH IV

worin $R^6$ niederes Alkanoyl oder Aryl-(niederes Alkyl) bedeutet,

umsetzt, oder

(c) zur Herstellung von Verbindungen der allgemeinen Formel I, worin $R^1$ Mercapto bedeutet, und A, $R^2$, $R^3$, $R^4$, $R^5$ und die gestrichelte Linie obige Bedeutung besitzen, in einer Verbindung der allgemeinen Formel

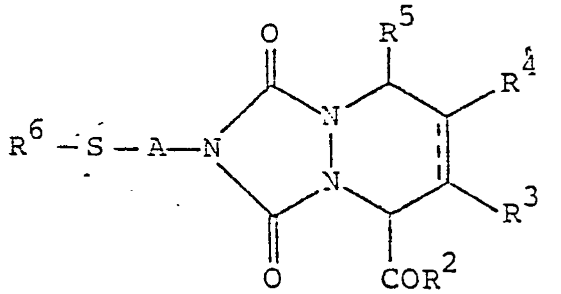

Ib

worin A, $R^2$, $R^5$, $R^6$ und die gestrichelte Linie obige Bedeutung besitzen,

die mit $R^6$ bezeichnete Gruppe abspaltet, oder

(d) zur Herstellung von Verbindungen der allgemeinen Formel I, worin $R^1$ Aminocarbonyl, Hydroxyaminocarbonyl oder Hydrazinocarbonyl bedeutet, und A, $R^2$, $R^3$, $R^4$, $R^5$ und die gestrichelte Linie obige Bedeutung besitzen, eine Verbindung der allgemeinen Formel

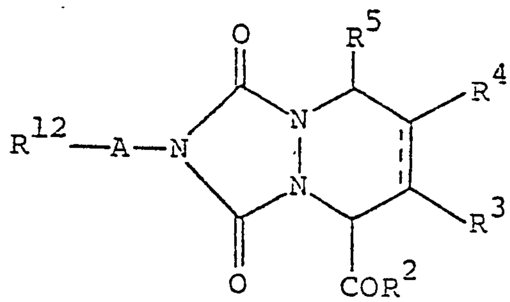

Ic

worin A, $R^2$, $R^3$, $R^4$, $R^5$ und die gestrichelte Linie obige Bedeutung besitzen, und $R^{12}$ niederes Alkoxy-

carbonyl bedeutet,
mit einer Verbindung der allgemeinen Formel

$$Y\text{-}NH_2 \qquad V$$

worin Y Wasserstoff, Hydroxy oder Amino bedeutet,
umsetzt, oder

(e) zur Herstellung von Verbindungen der allgemeinen Formel I, worin $R^1$ Halogen, Carboxyl, niederes Alkoxycarbonyl, Aminocarbonyl, Hydroxyaminocarbonyl oder Hydrazinocarbonyl, $R^3$ und $R^4$ beide Wasserstoff bedeuten, in 6,7-Stellung eine Einfachbindung vorhanden ist, und A, $R^2$ und $R^5$ obige Bedeutung besitzen, eine entsprechende Verbindung der allgemeinen Formel I, worin in 6,7-Stellung eine Doppelbindung vorhanden ist, katalytisch hydriert, oder

(f) eine Verbindung der allgemeinen Formel

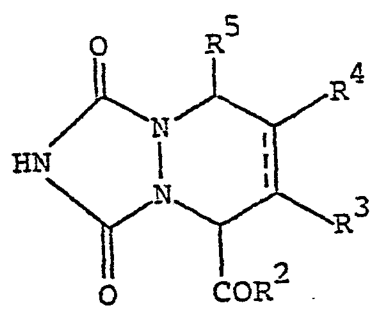

VI.

worin $R^2$, $R^3$, $R^4$, $R^5$ und die gestrichelte Linie obige Bedeutung besitzen,
mit einer Verbindung der allgemeinen Formel

$$R^1\text{-}A\text{-}X \qquad VII$$

worin $R^1$ und A obige Bedeutung besitzen und X eine Abgangsgruppe bedeutet,
umsetzt, oder

(g) zur Herstellung von Verbindungen der allgemeinen

Formel I, worin A eine Methylen-Gruppe, $R^1$ Halogen und $R^2$ niederes Alkoxy oder Amino bedeuten und $R^3$, $R^4$, $R^5$ und die gestrichelte Linie obige Bedeutung besitzen, eine Verbindung der allgemeinen Formel

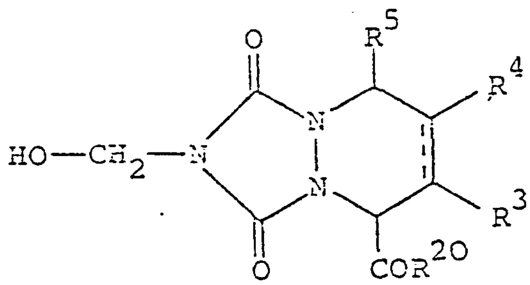

VIII

worin $R^3$, $R^4$, $R^5$ und die gestrichelte Linie obige Bedeutung besitzen, und $R^{20}$ niederes Alkoxy oder Amino bedeutet,

geeignet halogeniert, oder

(h) zur Herstellung von Verbindungen der allgemeinen Formel I, worin $R^3$ und $R^4$ beide Halogen bedeuten, in 6,7-Stellung eine Einfachbindung vorhanden ist, und A, $R^1$, $R^2$ und $R^5$ obige Bedeutung besitzen, eine Verbindung der allgemeinen Formel I, worin in 6,7-Stellung eine Doppelbindung vorhanden ist, mit einem Halogenwasserstoff in Gegenwart von Wasserstoffperoxid behandelt, oder

(i) zur Herstellung von Verbindungen der allgemeinen Formel I, worin $R^3$ Wasserstoff und $R^4$ Hydroxy bedeuten, in 6,7-Stellung eine Einfachbindung vorhanden ist, und A, $R^1$, $R^2$ und $R^5$ obige Bedeutung besitzen, eine Verbindung der allgemeinen Formel

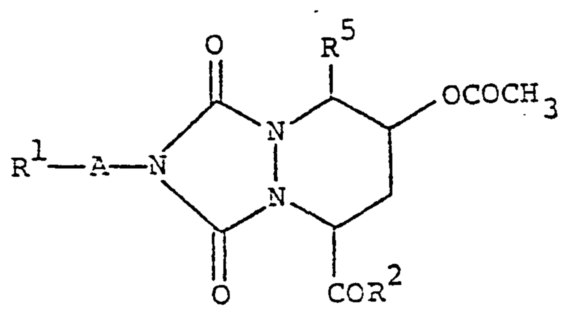

XI

worin A, $R^1$, $R^2$ und $R^5$ obige Bedeutung besitzen, hydrolysiert, oder

(j) zur Herstellung von Verbindungen der allgemeinen Formel I, worin $R^1$ niederes Alkoxycarbonyl und/oder $R^2$ niederes Alkoxy bedeutet, eine entsprechende Verbindung der allgemeinen Formel I, worin $R^1$ Carboxyl und/oder $R^2$ Hydroxy bedeuten, verestert, oder

(k) zur Herstellung von Verbindungen der allgemeinen Formel I, worin $R^1$ Carboxyl und/oder $R^2$ Hydroxy bedeutet, eine entsprechende Verbindung der allgemeinen Formel I, worin $R^1$ niederes Alkoxycarbonyl und/oder $R^2$ niederes Alkoxy bedeutet, mit einer Säure oder einer Base behandelt, oder

(l) zur Herstellung eines Salzes einer Carbonsäure der allgemeinen Formel I, eine Carbonsäure der allgemeinen Formel I in ein pharmazeutisch annehmbares Salz mit einer Base überführt.

2. Verfahren gemäss Anspruch 1, dadurch gekennzeichnet, dass A eine Aethylen-Gruppe bedeutet.

3. Verfahren gemäss Anspruch 1 oder 2, dadurch gekennzeichnet, dass $R^1$ Carboxyl, Hydroxyaminocarbonyl oder Mercapto bedeutet.

4. Verfahren gemäss einem der Ansprüche 1 bis 3, dadurch gekennzeichnet, dass $R^2$ Hydroxy bedeutet.

5. Verfahren gemäss einem der Ansprüche 1 bis 4, dadurch gekennzeichnet, dass in 6,7-Stellung eine Doppelbindung vorhanden ist.

6. Verfahren gemäss einem der Ansprüche 1 bis 5, dadurch gekennzeichnet, dass $R^4$ Wasserstoff oder Aryl bedeutet.

7. Verfahren gemäss Anspruch 1, dadurch gekennzeichnet, dass man 2,3,5,8-Tetrahydro-2-(2-mercaptoäthyl)-1,3-dioxo-1H-1,2,4-triazolo[1,2-a]pyridazin-5-carbonsäure herstellt.

8. Verfahren gemäss Anspruch 1, dadurch gekennzeichnet, dass man 5-Carboxy-2,3,5,8-tetrahydro-1,3-dioxo-1H-1,2,4-triazolo[1,2-a]pyridazin-2-propionsäure herstellt.

9. Verfahren gemäss Anspruch 1, dadurch gekennzeichnet, dass man 2-[2-(N-Hydroxycarbamoyl)äthyl]-2,3,5,8-tetrahydro-1,3-dioxo-1H-1,2,4-triazolo[1,2-a]pyridazin-5-carbonsäure herstellt.

10. Verfahren gemäss Anspruch 1, dadurch gekennzeichnet, dass man 5-Carboxy-2,3,5,8-tetrahydro-8-phenyl-1,3-dioxo-1H-1,2,4-triazolo[1,2-a]pyridazin-2-propionsäure herstellt.